# EUROPEAN PATENT SPECIFICATION

(11) **EP 2 818 118 B1**
(45) Date of publication and mention of the grant of the patent: **20.09.2017**
(21) Application number: 14174062.1
(22) Date of filing: 26.06.2014
(51) Int. Cl.: A61B 8/00, G06F 3/01

(54) **Method of moving the displays of an ultrasound diagnostic device and ultrasound diagnostic device**
Verfahren zum Bewegen der Anzeigen einer Ultraschalldiagnosevorrichtung sowie Ultraschalldiagnosevorrichtung
Procédé permettant de déplacer les affichages d'un dispositif de diagnostic à ultrasons et ledit dispositif

(30) Priority: 28.06.2013 KR 20130075944
(43) Date of publication of application: 31.12.2014
(73) Proprietor: Samsung Electronics Co., Ltd., Gyeonggi-do 443-742 (KR)
(72) Inventor: Han, Jeong-Ho, Seoul (KR); Yang, Eun-ho, Seoul (KR)
(74) Representative: Grootscholten, Johannes A.M.

(56) References cited:
- EP-A1- 2 458 472
- KR-A- 20100 052 005
- US-A1- 2007 276 244
- US-A1- 2012 075 166
- US-A1- 2013 093 738

## Description

### BACKGROUND

### 1. Field

Exemplary embodiments relate to a method of moving a display of an ultrasound diagnostic device and to an ultrasound diagnostic device, and more particularly, to a method of moving a display of an ultrasound diagnostic device based on an operator's viewing direction, and to an ultrasound diagnostic device.

### 2. Description of the Related Art

An ultrasound diagnostic device is used to observe the internal structure of an organic body. The ultrasound diagnostic device is non-invasive testing equipment that shows structural detailed items, internal tissues, and flow of a liquid in a human body.

The ultrasound diagnostic device irradiates an ultrasound signal generated from a transducer of a probe to an object and receives information of a response signal reflected from the object, thereby obtaining an image of the interior of the object. In particular, the ultrasound diagnostic device is used for a medical purpose such as observation of the interior of an object, detection of foreign materials, and measurement of an injury, etc.

The ultrasound diagnostic device exhibits high stability. Further, the ultrasound diagnostic methods provide more safety than other imaging methods, such as X-ray and CT imaging methods, due to the fact that ultrasound diagnostic methods do not involve exposure to hazardous radiation such as X-rays. Also, since real-time image display is possible, the ultrasound diagnostic device is widely used with other imaging diagnostic devices.

In addition or alternatively for the above indication of the prior art in relation to the present invention, reference is made here to the prior art disclosure in EP-2458472, relative to which at least the features in the characterizing portion of the appended independent claims are novel. Similarly, the prior art disclosure of US-2007/276244 is acknowledged here, but the content/disclosure thereof is even more remote from the subject matter of the present invention.

### SUMMARY OF THE INVENTION

Exemplary embodiments of the present invention provide a method of moving a display of an ultrasound diagnostic device, and an ultrasound diagnostic device, which enables an operator who uses the ultrasound diagnostic device to operate a probe and simultaneously easily check a display. The invention is defined by the appended claims.

According to a first exemplary embodiment is provided a method of operating a display of an ultrasound diagnostic device having at least one display, the method comprising:
obtaining an image of an operator of the ultrasound diagnostic device performing ultrasound imaging of an object by using the ultrasound diagnostic device; determining at least a viewing direction and optionally a position of the operator from the obtained image of the operator; and
changing at least one of an orientation from a first angle to a second angle, and a position from a first position to a second position of the display connected to the ultrasound diagnostic device according to at least the determined viewing direction and optionally the determined position of the operator, wherein the image of the operator further comprises a support table supporting the object, and the determining of at least the viewing direction and/or the determining of the position of the operator comprises determining a positional relationship between the operator and the support table comprised in the image of the operator, including the support table supporting the object.

The changing of the position of the display may include determining a visible range of the operator based on the position and the viewing direction of the operator, and changing the position of the display from the first position to the second position so that a screen of the display is located in the determined visible range of the operator.

The determining of the viewing direction of the operator may include determining a posture of the operator based on the image of the operator, and determining the viewing direction of the operator by using the determined posture of the operator.

The determining of the posture of the operator may include determining the posture of the operator based on at least one of a shape and a positional relationship of a head part and a body part of the operator included in the image of the operator.

The changing of the position of the display may include determining the second position in consideration of a positional relationship between the ultrasound diagnostic device and the operator.

The changing of the position of the display may include determining a position located in the visible range of the operator in a space within which the display is movable, as the second position.

The obtaining of the image of the operator may include obtaining a depth image of the operator, and the determining of the position and the viewing direction of the operator comprises determining the position and the viewing direction of the operator based on the depth image of the operator.

The obtaining of the depth image may include obtaining a depth image of the operator by using a depth camera.

The obtaining of the depth image may include obtaining images of the operator by using at least two color cameras, and obtaining a depth image of the operator by applying stereo matching to the images of the operator obtained by using the at least two color cameras.

The changing of the position of the display includes determining a movement path from the first position to the second position based on information about a position of at least one of a person and an object included in the image of the operator.

The determining of the movement path may include determining the movement path from the first position to the second position to guide the display not to collide against at least one of the person and the object included in the image of the operator.

The changing of the position of the display may include changing a movement path of the display when it is determined that an obstacle exists on the movement path of the display while the position of the display is changed from the first position to the second position.

The method may further include obtaining information about a portion of the object, obtaining an image of the object, determining a position of a portion corresponding to the portion in the image of the object, and changing a position of the display from a third position to the first position according to the determined position of the portion.

The obtaining of the image of the object may include obtaining a depth image of the object.

The determining of the position and the viewing direction of the operator may include determining a person who is the closest to a position of a probe of the ultrasound diagnostic device when a plurality of persons are included in the image of the operator.

The determining of the person who is the closest to the position of the probe of the ultrasound diagnostic device as the operator may include determining the position of the probe by using infrared or short-rang communication, and determining a person who is the closest to the position of the probe based on the position of the probe and the image of the operator, as the operator.

The display may include a first display and a second display, and the method further comprising adjusting an angle between the first display and the second display.

The adjusting of the angle between the first display and the second display may include adjusting the angle between the first display and the second display so that a screen of the first display and a screen of the second display are located at the viewing direction of the operator.

The adjusting of the angle between the first display and the second display may include adjusting the angle between the first display and the second display so that a screen of the first display is located at the viewing direction of the operator and the screen of the second display is located at a viewing direction of the object.

The method may further include adjusting at least one of a brightness value, a contrast value, and a tilting angle of the display located at the second position according to a preset value.

The method may further include detecting gesture of the operator with respect to the display located at the second position, and performing a function of the ultrasound diagnostic device corresponding to the gesture of the operator.

The detecting of the gesture of the operator may include detecting gesture of the operator by using the image of the operator.

The detecting of the gesture of the operator may include performing a function corresponding to a position indicated by a hand of the operator among functions displayed on the display when it is determined that the hand of the operator and the display are located within a preset distance for a preset time, based on the image of the operator.

The detecting of the gesture of the operator may include detecting gesture of the operator by using a gesture detection camera attached to the display.

The detecting of the gesture of the operator may include detecting gesture of an eye of the operator by using an eye tracking sensor attached to the display, and performing a function of the ultrasound diagnostic device corresponding to the detected gesture of the eye of the operator.

The method may further include receiving a display position change input by the operator.

The display position change input may include at least one of an input through a foot switch connected to the ultrasound diagnostic device, an input through operator's voice, and an input through operator's gesture.

The method may further includes receiving a capturing end input by the operator, and changing the position of the display from the second position to the first position according to the capturing end input.

According to another exemplary embodiment, a method of moving a display of an ultrasound diagnostic device includes obtaining an image of an object whose ultrasound image is to be captured by using the ultrasound diagnostic device, obtaining information about a portion of the object, determining a position of a portion corresponding to the portion based on the image of the object, and changing a position of the display connected to the ultrasound diagnostic device from a first position to a second position according to the determined position of the portion.

According to another exemplary embodiment, a method of moving a display of an ultrasound diagnostic device includes obtaining an image of an operator who captures an ultrasound image of an object by using the ultrasound diagnostic device, i.e. operates the ultrasonic device to capture the ultrasonic images, determining a viewing direction of the operator from the obtained image of the operator, and adjusting an angle between a first display and a second display that are connected to the ultrasound diagnostic device, according to the viewing direction of the operator.

The adjusting of the angle between the first display and the second display may include adjusting the angle between the first display and the second display so that a screen of the first display and a screen of the second display are located at the viewing direction of the operator.

The method may further include obtaining an image of the object, and determining a viewing direction of the object based on the image of the object, wherein the adjusting of the angle between the first display and the second display includes adjusting the angle between the first display and the second display so that a screen of the first display is located at the viewing direction of the operator and the screen of the second display is located at a viewing direction of the object.

According to another exemplary embodiment, there is provided a non-transitory computer readable recording medium having recorded thereon a program for executing the above method.

According to another exemplary embodiment, an ultrasound diagnostic device includes an image obtaining unit obtaining an image of an operator capturing an ultrasound image of an object, a determination unit determining a position and a viewing direction of the operator based on the image of the operator, a display displaying at least one of information of the object and an ultrasound image of the object, and a control unit changing a position of the display from a first position to a second position according to the position and the viewing direction of the operator.

The determination unit may determine a visible range of the operator based on the position and the viewing direction of the operator, and the control unit may change the position of the display from the first position to the second position so that a screen of the display is located within the visible range of the operator.

The determination unit may determine a posture of the operator based on the image of the operator and determines the viewing direction of the operator by using the determined posture of the operator.

The determination unit may determine the posture of the operator based on at least one of a shape and a positional relationship of a head part and a body part of the operator included in the image of the operator.

The image of the operator may include a support table supporting the object, and the determination unit may determine the viewing direction of the operator in consideration of a positional relationship between the operator and the support table included in the image of the operator.

The control unit may determine the second position in consideration of a positional relationship between the ultrasound diagnostic device and the operator.

The control unit may determine a position located in the visible range of the operator in a space within which the display is movable, as the second position.

The image obtaining unit may obtain a depth image of the operator, and the determination unit may determine the position and the viewing direction of the operator based on the depth image of the operator.

The image obtaining unit may include a depth camera for obtaining a depth image of the operator.

The image obtaining unit may include at least two color cameras for obtaining images of the operator and obtain a depth image of the operator by applying stereo matching to the images of the operator obtained by using the at least two color cameras.

The control unit may determine a movement path from the first position to the second position based on information about a position of at least one of a person and an object included in the image of the operator.

The control unit may determine the movement path from the first position to the second position to guide the display not to collide against at least one of the person and the object included in the image of the operator.

The display may include a distance detection sensor that detects an obstacle located within a predetermined distance.

The control unit may change the movement path of the display when an obstacle detected by the distance detection sensor exists on the movement path of the display while the position of the display is changed from the first position to the second position.

The ultrasound diagnostic device may further include a communication unit obtaining information about a portion of the object, wherein the image obtaining unit obtains an image of the object, the determination unit may determine a position of a portion corresponding to the portion in the image of the object, and the control unit may change a position of the display from a third position to the first position according to a determined position of the portion.

The determination unit may determine a person who is the closest to a position of a probe of the ultrasound diagnostic device when a plurality of persons are included in the image of the operator.

The ultrasound diagnostic device may further include an infrared emitting unit that emits an infrared ray, in which the determination unit determines a position of an object that reflects or absorbs the infrared ray emitted by the infrared emitting unit, as a position of the probe.

The display may include a first display and a second display, and the control unit may adjust an angle between the first display and the second display.

The control unit may adjust the angle between the first display and the second display so that a screen of the first display and a screen of the second display are located at the viewing direction of the operator.

The control unit may adjust the angle between the first display and the second display so that a screen of the first display is located at the viewing direction of the operator and the screen of the second display is located at a viewing direction of the object.

The control unit may adjust at least one of a brightness value, a contrast value, and a tilting angle of the display located at the second position according to a preset value.

The ultrasound diagnostic device may further include a gesture detection unit that detects gesture of the operator with respect to the display located at the second position, in which the control unit performs a function of the ultrasound diagnostic device corresponding to the gesture of the operator.

The gesture detection unit may detect gesture of the operator by using the image of the operator.

The gesture detection unit may determine whether a hand of the operator and the display are located within a preset distance for a preset time, based on the image of the operator, and the control unit may perform a function corresponding to a position indicated by the hand of the operator among functions displayed on the display.

The gesture detection unit may include a gesture detection camera that is attached on the display and detects gesture of the operator.

The gesture detection unit may include an eye tracking sensor that is attached to the display and detects gesture of an eye of the operator, and the control unit may perform a function of the ultrasound diagnostic device corresponding to the detected gesture of the eye of the operator.

The ultrasound diagnostic device may further include a communication unit that receives a display position change input by the operator.

The display position change input may include at least one of an input through a foot switch connected to the ultrasound diagnostic device, an input through operator's voice, and an input through operator's gesture.

The ultrasound diagnostic device may further include a communication unit that receives a capturing end input by the operator, in which the control unit changes the position of the display from the second position to the first position according to the capturing end input.

According to exemplary embodiments, an ultrasound diagnostic device includes an image obtaining unit obtaining an image of an object whose ultrasound image is to be captured, a communication unit obtaining information about a portion of the object, a determination unit determining a position of a portion corresponding to the portion based on the image of the object, a display displaying at least one of information of the object and an ultrasound image of the object, and a control unit changing a position of the display from a first position to a second position according to the determined position of the portion.

According to another exemplary embodiment, an ultrasound diagnostic device includes an image obtaining unit obtaining an image of an operator who captures an ultrasound image of an object, a determination unit determining a viewing direction of the operator from the image of the operator, a first display and a second display displaying at least one of information of the object and the ultrasound image of the object, and a control unit adjusting an angle between the first display and the second display that are connected to the ultrasound diagnostic device, according to the viewing direction of the operator.

The control unit may adjust the angle between the first display and the second display so that a screen of the first display and a screen of the second display are located at the viewing direction of the operator.

The image obtaining unit may obtain an image of the object, the determination unit may determine a viewing direction of the object based on the image of the object, and the control unit may adjust the angle between the first display and the second display so that a screen of the first display is located at the viewing direction of the operator and the screen of the second display is located at a viewing direction of the object.
The image obtaining unit may obtain a depth image of the operator, and the determination unit may determine the viewing direction of the operator from the depth image of the operator.

The foregoing general description and the following detailed description are only exemplary and explanatory and they are intended to provide further explanation of the invention as claimed.

### BRIEF DESCRIPTION OF THE DRAWINGS

The above and other features and advantages of the exemplary embodiments will become more apparent by describing in detail exemplary embodiments thereof with reference to the attached drawings in which:
FIG. 1 illustrates an environment in which an operator operates a general ultrasound diagnostic device;
FIG. 2 illustrates the operation of an ultrasound diagnostic device according to an exemplary embodiment;
FIG. 3 is a flowchart for explaining a method of moving a display of an ultrasound diagnostic device, according to an exemplary embodiment;
FIG. 4 is a flowchart for explaining Operation S310 of FIG. 3 in detail;
FIG. 5 illustrates a method of determining an operator from a depth image including a plurality of persons;
FIG. 6 is a flowchart for explaining Operation S320 of FIG. 3 in detail;
FIG. 7A illustrates a depth image;
FIG. 7B illustrates a method of determining a viewing direction of a person included in the depth image;
FIG. 8 illustrates another method of determining a viewing direction of an operator from a depth image;
FIG. 9 illustrates another method of determining a viewing direction of an operator from a depth image;
FIG. 10 is a flowchart for explaining Operation S330 of FIG. 3 in detail;
FIG. 11 illustrates a method of determining a second position considering a positional relationship between an ultrasound diagnostic device and an operator;
FIG. 12 illustrates a method of determining a movement path between a first position and the second position from a depth image;
FIG. 13 is a flowchart for explaining a method of moving a display of an ultrasound diagnostic device, according to another exemplary embodiment;
FIG. 14 illustrates a method of moving a display from a third position to the first position;
FIG. 15 is a flowchart for explaining a method of moving a display of an ultrasound diagnostic device, according to another exemplary embodiment;
FIG. 16 is a flowchart for explaining a method of moving a display of an ultrasound diagnostic device, according to another exemplary embodiment;
FIG. 17 illustrates a method of adjusting an angle between a first display and a second display;
FIG. 18 illustrates another method of adjusting an angle between the first display and the second display;
FIG. 19A illustrates a method of detecting gesture of an operator from a depth image;
FIG. 19B illustrates a method of performing a function of an ultrasound diagnostic device displayed on a display according to operator's gesture;
FIG. 20 is a block diagram illustrating the structure of an ultrasound diagnostic device according to an exemplary embodiment;
FIG. 21 is a block diagram illustrating the structure of an ultrasound diagnostic device according to another exemplary embodiment; and
FIG. 22 is a block diagram illustrating the structure of a wireless probe that may be connected to an ultrasound diagnostic device, according to an exemplary embodiment.

### DETAILED DESCRIPTION

The following detailed description is provided to gain a comprehensive understanding of the methods, apparatuses and systems described herein. Various changes, modifications, and equivalents of the systems, apparatuses and methods described herein will suggest themselves to those of ordinary skill in the art. Descriptions of well-known functions and structures are omitted to enhance clarity and conciseness.

The attached drawings for illustrating exemplary embodiments of the present invention are referred to in order to gain a sufficient understanding of the present invention, the merits thereof, and the objectives accomplished by the implementation of the present invention. Hereinafter, the present invention will be described in detail by explaining exemplary embodiments of the invention with reference to the attached drawings. Like reference numerals in the drawings denote like elements.

Terms used in the present specification will be briefly described and the present invention will be described in detail. The terminology used herein is for the purpose of describing particular embodiments only and is not intended to be limiting of the present disclosure.

General terms that are currently widely used are selected as the terms used for the present invention in consideration of functionality in the present invention and availability. However, the terms may vary according to intention of engineers working in the present technical field, precedents, advent of new technology, etc. Also, in a specific case, the applicant particularly selects terms and in such a case the meaning of a term is defined in detail in the detailed description section. Accordingly, the terms used for the present invention should be defined based on the meaning of the term and the overall context of the present specification, not simply by a nominal meaning of the term.

When a part may "include" a certain constituent element, unless specified otherwise, it may not be construed to exclude another constituent element but may be construed to further include other constituent elements. The terms such as "portion", "unit", "module", and "block" stated in the specification may signify a unit to process at least one function or operation and the unit may be embodied by hardware such as FPGA or ASIC, software, or a combination of hardware and software. However, the term "portion" in the present invention is not limited by a specific combination of hardware and software. The "portion" may be configured in a storage medium that may be addressed or to be able to reproduce one or more processors. Accordingly, as an example, the "portion" includes constituent elements such as software constituent elements, object-oriented software constituent elements, class constituent elements, and task constitute elements, processes, functions, attributes, procedures, subroutines, segments of a program code, drivers, firmware, micro-codes, circuits, data, database, data structures, tables, arrays, and variables. Functions provided in the constituent elements and the "portions" may be combined into a smaller number of constituent elements or the "portions" or classified into additional constituent elements and the "portions".

As used herein, the term "and/or" includes any and all combinations of one or more of the associated listed items. Expressions such as "at least one of," when preceding a list of elements, modify the entire list of elements and do not modify the individual elements of the list. Although some features may be described with respect to individual exemplary embodiments, aspects need not be limited thereto such that features from one or more exemplary embodiments may be combinable with other features from one or more exemplary embodiments.

In the present specification, an "image" may signify multi-dimensional data formed of discrete image elements (for example, pixels in a two-dimensional image and voxels in a three-dimensional image). For example, the image may include a medical image of an object obtained by X-ray, CT, MRI, ultrasound, and other medical imaging systems.

Also, in the present specification, an "object" may include the body of a human or an animal, or a part thereof. For example, the object may include internal organs such as liver, heart, womb, brain, breasts, abdomen, etc. or blood vessels. Also, the "object" may include phantom. The phantom may signify a substance having a density of a living thing and a volume closely approximate to the effective atom number and may include spherical phantom having a similar feature to a human body.

Also, in the present specification, a "operator" may be a medical expert, for example, a medical doctor, a nurse, a clinical technologist, a medical imaging expert, etc. and a technologist who fixes medical equipment, but the exemplary embodiments are not limited thereto.

Also, in the present specification, an "ultrasound image" signifies an image about an object obtained by using an ultrasonic wave.

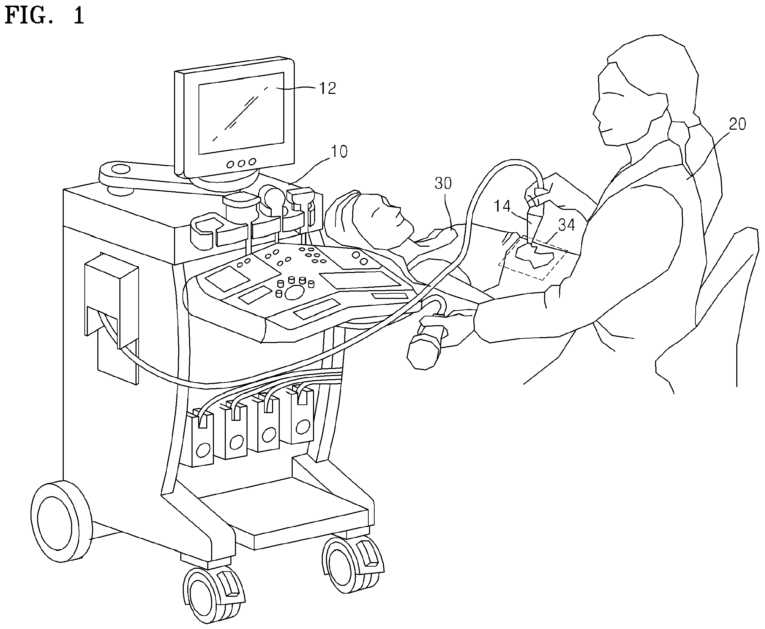

FIG. 1 illustrates an environment in which an operator 20 operates a general ultrasound diagnostic device 10. Referring to FIG. 1, the general ultrasound diagnostic device 10 includes a display 12 displaying an ultrasound image of an object 30 and a probe 14 transmitting an ultrasound signal to the object 30 and receiving a response signal reflected from the object 30. The operator 20 locates the probe 14 to a portion 34 of the object 30 to obtain an image of the interior of the object 30 and observes an ultrasound image displayed on the display 12.

In the general ultrasound diagnostic device 10, since the display 12 is fixedly attached on the general ultrasound diagnostic device 10, if the portion 34 of the object 30 and the display 12 of the general ultrasound diagnostic device 10 are located in different directions, the operator 20 needs to continuously change a viewing direction between the portion 34 of the object 30 and a screen of the display 12. This may be very inconvenient for the operator 20 to diagnose the object 30.

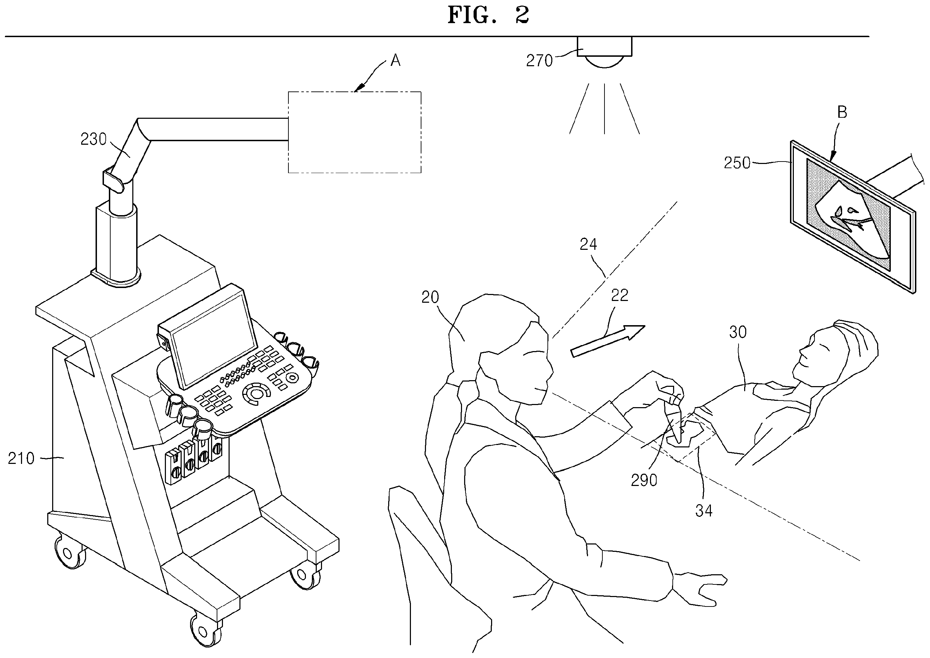

FIG. 2 illustrates the operation of an ultrasound diagnostic device 210 according to an exemplary embodiment. The ultrasound diagnostic device 210 according to the present embodiment determines a viewing direction 22 of the operator 20 based on an image of the operator 20 obtained by a black and white camera, a depth camera, or a color camera 270. Next, the ultrasound diagnostic device 210 may change the position of a display 250 according to the determined viewing direction 22 from a first position A to a second position B.

The ultrasound diagnostic device 210 and the display 250 may be connected by a multi-joint arm 230. The multi-joint arm 230 may have a degree of freedom of at least 6 axes and may move the display 250 in a direction and to a position that the operator 20 desires. The structure of the multi-joint arm 230 in FIG. 2 is an example and the ultrasound diagnostic device 210 may comprise an arm having various structures that may move the position of the display 250.

Referring to FIG. 2, the display 250 of the ultrasound diagnostic device 210 is moved from the first position A to the second position B and thus the portion 34 of the object 30 and a screen of the display 250 both may be located within a visible range 24 of the operator 20. Accordingly, the operator 20 may operate a probe 290 and simultaneously easily check the screen of the display 250.

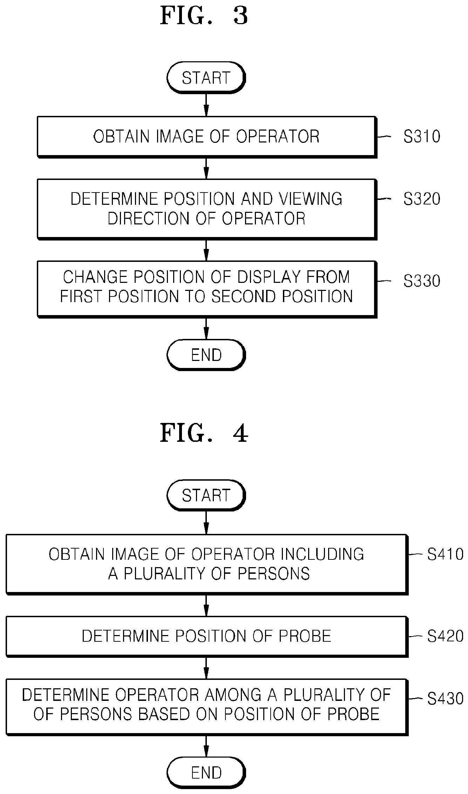

FIG. 3 is a flowchart for explaining a method of moving a display of an ultrasound diagnostic device, according to an exemplary embodiment. Referring to FIG. 3, in Operation S310, the ultrasound diagnostic device 210 obtains an image of an operator. The ultrasound diagnostic device 210 may continuously obtain images of an operator at a predetermined time interval.

The image of an operator is obtained by using a color camera, a black and white camera, or a depth camera. Also, the image of an operator may include a depth image of an operator. The depth image signifies an image including information about a distance between a camera capturing a depth image and an object captured by the camera. The images may be obtained by a plurality of cameras disposed at various positions in the ultrasound diagnostic room. For example, cameras may be disposed on the walls, ceiling, or stands (the stands may be used to adjust the positions of the camera). Further, cameras may be attached to specific parts of the ultrasound diagnostic apparatus. For example, cameras may be attached or incorporated to a side of the displays.

The ultrasound diagnostic device 210 according to the present embodiment may receive an image of an operator from an external device or server or obtain an image of an operator by using a color camera, a black and white camera, or a depth camera.

Also, the ultrasound diagnostic device 210 may obtain a depth image of an operator by using a depth camera or obtain a depth image by applying stereo matching to images of an operator obtained from at least two color cameras.

A method of obtaining a depth image including information about a distance of an object by applying stereo matching to the image of an operator obtained by using at least two color cameras is well known to one of ordinary skill in the art and thus a detailed description thereof will be omitted in the present specification.

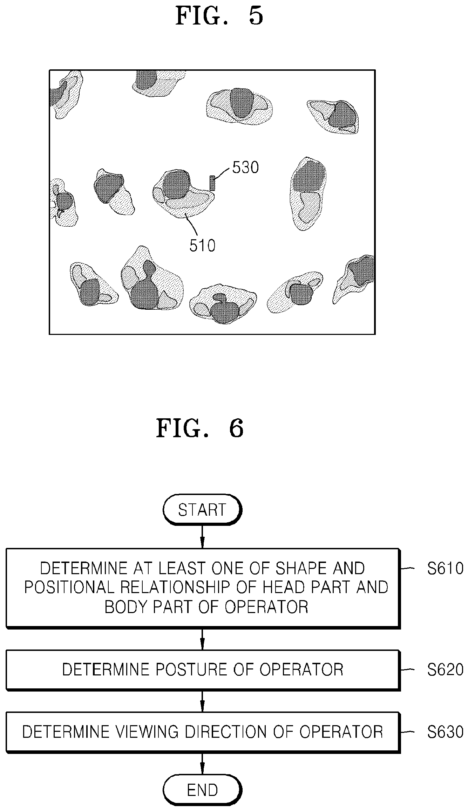

In Operation S320, the ultrasound diagnostic device 210 determines a position and a viewing direction of an operator from the image of an operator. In other words, the ultrasound diagnostic device 210 determines the position where the operator is located in a predetermined space including the operator and a viewing direction or a direction in which the operator looks. A method of determining a viewing direction of an operator is described below in detail with reference to FIGS. 6 to 9. A viewing direction may refer to a direction in which the operator looks, a viewing field, or a field of view.

In Operation S330, the ultrasound diagnostic device 210 changes the position of the display 250 from a first position to a second position according to the position and viewing direction of an operator. In detail, the ultrasound diagnostic device 210 may determine a position to which the display 250 moves considering the position and viewing direction of an operator and move the position of the display 250 to the determined position. The ultrasound diagnostic device 210 may change the position of the display 250 from the first position to the second position so that the screen of the display 250 may be located within a visible range of an operator. The display may be sequentially moved through various positions. The movement of the display between two positions, such as from a first position to a second position, may be performed on various paths.

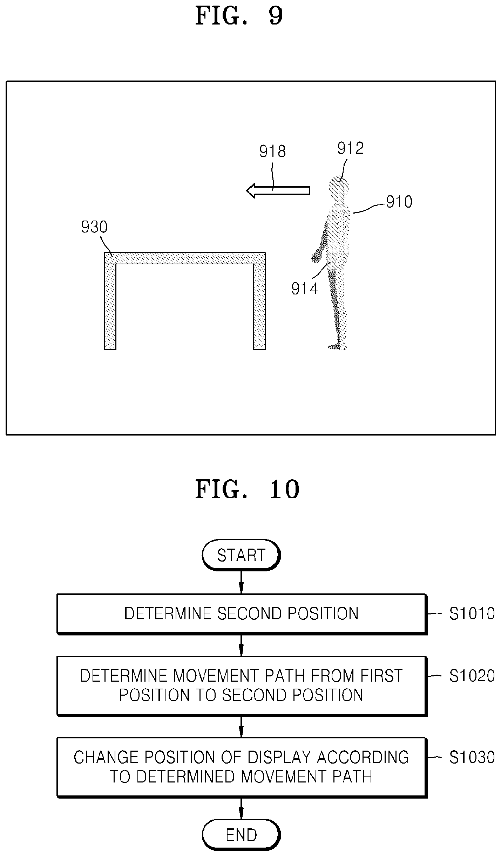

The ultrasound diagnostic device 210 may determine the second position considering a positional relationship between the operator and the ultrasound diagnostic device 210 or determine a movement path to prevent collision between the display 250 and other objects, which will be described below with reference to FIGS. 10 to 12.

When an operator takes a posture to easily observe the portion 34 of the object 30 at a certain position and the display 250 of the ultrasound diagnostic device 210 moves to the position of the operator and along the viewing direction of the operator, the portion 34 of the object 30 and the screen of the display 250 may be located altogether within the visible range of the operator. Accordingly, the operator may operate the probe 290 and simultaneously easily check the screen of the display 250.

The ultrasound diagnostic device 210 may adjust at least one of a brightness value, a contrast value, and a tilting angle of the display 250 located at the second position according to a preset value. When at least one of the brightness value, the contrast value, and the tilting angle of the display 250 is preset by an operator, at least one of the brightness value, the contrast value, and the tilting angle of the display 250 located at the second position is adjusted according to the preset value and thus operator convenience may be improved.

FIG. 4 is a flowchart for explaining Operation S310 of FIG. 3 in detail. In detail, FIG. 4 is a flowchart for explaining a method of determining an operator when a plurality of persons are included in an image of an operator.

In Operation S410, the ultrasound diagnostic device 210 obtains an image of an operator. The image may include a plurality of persons. As described above, the image of an operator may be received from an external device or server or may be obtained from a color camera, a black and white camera, or a depth camera.

In Operation S420, the ultrasound diagnostic device 210 determined the position of the probe 290 from the image of an operator. The ultrasound diagnostic device 210 may determine the position of the probe 290 in a variety of methods. For example, the ultrasound diagnostic device 210 may determine the position of the probe 290 by identifying the probe 290 from the image of an operator. When the probe 290 may not be identified from the image of an operator, the ultrasound diagnostic device 210 may determine the position of the probe 290 in a predetermined space by using an infrared ray that is emitted into the predetermined space and detecting a reflected or absorbed infrared ray. An infrared reflection portion for reflecting an infrared ray or an infrared absorption portion for absorbing an infrared ray may be attached on the probe 290. Also, the ultrasound diagnostic device 210 may determined the position of the probe 290 by using short range communication such as RFID, Bluetooth, etc. Alternatively, a particular color or shape is marked on the probe 290 and then the color or shape of the probe 290 is identified by using a color camera, thereby determining the position of the probe 290. However, the above methods for determining the position of the probe and for identifying the probe may be applied to other objects in the room where the ultrasound diagnostic device is located. The determined positions and identities of the probe and the other objects may be used such as to ensure that the displays do not collide with the objects when moving from one position to the next.

In Operation S430, the ultrasound diagnostic device 210 determines as an operator a person who is the closest to the position of the probe 290 among the persons included in the image of an operator. Since an operator using the ultrasound diagnostic device 210 may possesses the probe 290, the ultrasound diagnostic device 210 determines as the operator a person who is the closest to the position of the probe 290 among the persons included in the image of an operator. When the ultrasound diagnostic device 210 determines the position of the probe 290 by using an infrared ray, short range communication, etc., the position of the probe 290 in the predetermined space where the probe 290 is located is mapped with the image of an operator and a person who is the closest to the position of the probe 290 may be determined to be the operator.

However, the methods of identifying an operator among the persons in the image are not limited to the above described methods. For example, similar to the method of identifying the probe 290, an operator may wear a particular mark or tag that the ultrasound diagnostic device 210 may recognize via an image recognition software. The operator may be identified, among the persons included in the images, by identifying which person in the images wears the particular mark or tag. The mark or tag may be worn as a separate effect or piece on operator's clothes, body parts, eyeglasses, headset or the like. The mark or tag may be painted (using for example a particular shape, color or fluorescence) on operator's clothes, body parts, eyeglasses, headset or the like. Similarly, other persons and images in the image may have or wear identifying marks and tags. For instance, an assistant of the operator may wear another tag or mark. This way the ultrasound diagnostic device 210 may identify various persons and objects in the images. Another method for identifying an operator or for facilitating the identification of the operator among the persons included in the images may include identifying which person in the images performs a certain gesture. For instance, at the start of the diagnostic process the operator may perform a specific hand gesture (e.g. snap his or her fingers) that an image recognition software may recognize in the images recorded by the camera.

FIG. 5 illustrates a method of determining an operator from a depth image including a plurality of persons. FIG. 5 illustrates a depth image of an operator included in a plurality of persons.

The ultrasound diagnostic device 210 may identify a probe 530 in an image of an operator including a plurality of persons and determine that a person 510 who is the closest to the position of the identified probe 530 as an operator.

FIG. 6 is a flowchart for explaining Operation S320 of FIG. 3 in detail. FIG. 6 illustrates a sequence in a method of determining a viewing direction of an operator.

In Operation S610, the ultrasound diagnostic device 210 determines at least one of a shape and a positional relationship of a head part and a body part from the image of an operator. In other words, the ultrasound diagnostic device 210 may identify a head part and a body part of an operator from the image of an operator and determine at least one of the shapes of the head part and the body part that are identified and a positional relationship between the head part and the body part.

In Operation S620, the ultrasound diagnostic device 210 determines the posture of an operator based on at least one of the shapes and the positional relationship of the head part and the body part of an operator. In other words, the ultrasound diagnostic device 210 may determine whether the operator is sitting, standing, or facing a certain direction considering at least one of the shapes and the positional relationship of the head part and the body part of an operator. A method of determining the posture of an operator by using at least one of the shapes and the positional relationship of the head part and the body part of the operator is a well-known technology to one of ordinary skill in the art and thus a detailed description thereof will be omitted herein.

In Operation S630, the ultrasound diagnostic device 210 determines the viewing direction of the operator based on the posture of the operator.

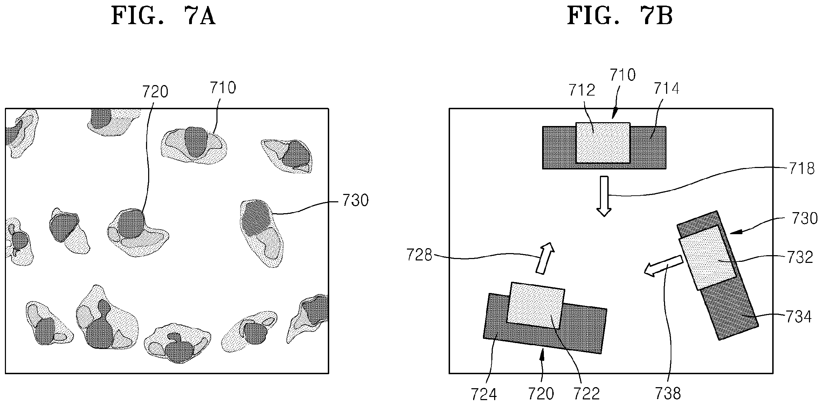

FIG. 7A illustrates a depth image and FIG. 7B illustrates a method of determining a viewing direction of a person included in the depth image.

Referring to FIG. 7A, the ultrasound diagnostic device 210 may identify head parts and body parts of persons included in a depth image by applying an image processing technique such as pattern matching, omega detection, adda boost, etc. to the depth image.

FIG. 7B schematically illustrates head parts 712, 722, and 732 and body parts 714, 724, and 734 of a first person 710, a second person 720, and a third person 730 included in the depth image of FIG. 7A. The ultrasound diagnostic device 210 may determine viewing directions 718, 728, and 738 of the first person 710, the second person 720, and the third person 730 by using at least one of the shapes and relative positional relationship of the head parts 712, 722, and 732 and the body parts 714, 724, and 734 of the first person 710, the second person 720, and the third person 730 of FIG. 7B. Referring to 7B, it can be seen that the viewing direction 718 of the first person 710, the viewing direction 728 of the second person 720, and the viewing direction 738 of the third person 730 are in the directions of six o'clock, one o'clock, and eight o'clock, respectively.

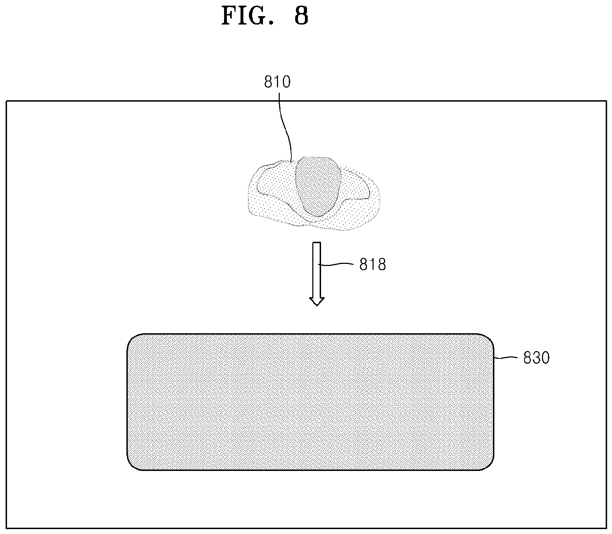

FIG. 8 illustrates another method of determining a viewing direction 818 of an operator 810 from a depth image. The depth image of the operator 810 may include a support table 830 supporting an object (not shown). Since the operator 810 takes a posture in a direction toward the object to diagnose the object, the viewing direction 818 of the operator 810 may be determined further considering a relative position between the operator 810 and the support table 830. Referring to FIG. 8, since the support table 830 is located in the direction of six o'clock of the operator 810 on the drawing sheet, the viewing direction 818 of the operator 810 may be determined to be in the direction of six o'clock.

FIG. 9 illustrates another method of determining a viewing direction 918 of an operator 910 from a depth image. While FIGS. 7 and 8 illustrate a depth image by capturing an operator from the top side, that is, from the ceiling, FIG. 9 illustrates a depth image captured from a lateral direction of the operator 910. A depth camera or a color camera capturing a depth image of an operator may be connected to a main body of the ultrasound diagnostic device 210 or located at a variety of positions within a range that is obvious to one of ordinary skill in the art.

For a depth image captured in the lateral direction, the viewing direction 918 of the operator 910 may be determined based on at least one of a shape and a relative positional relationship of a head part 912 and a body part 914 of the operator 910. Also, the ultrasound diagnostic device 210 may determined the viewing direction 918 of the operator 910 further considering a relative positional relationship between the operator 910 and a support table 930. Referring to FIG. 9, since the support table 930 is located in the direction of nine o'clock of the operator 910 on the drawing sheet, the viewing direction 918 of the operator 910 may be determined to be in the direction of nine o'clock. After determining the viewing direction of an operator the ultrasound diagnostic device 210 may determine whether the operator looks at the display or whether the operator looks at other objects such as the probe, the investigated person or object, or in other directions. The position of the display may be adjusted according to whether the operator looks towards the display or towards other objects.

In FIGS. 6-9, the viewing direction of an operator is described to be determined considering the shape and positional relationship of the head part and the body part of the operator. However, such a description is a mere example and the viewing direction of an operator may be determined by using various pieces of information such as the shape of a lower body of an operator , the position of a hand of an operator, the position of a foot of an operator, etc.

Further, a viewing direction of an operator may be found by using other techniques such as described in the following. In an exemplary embodiment, an eye tracking sensor may be used to track a direction indicated by the eyeballs of the operator, this way a viewing direction may be determined. The eye tracking sensor may be disposed in the room or on the operator's body (e.g. attached on a headset or eyeglasses).

In another exemplary embodiment, similar to a method of identifying the probe 290, the operator may wear or have one or more marks or tags attached to operator's clothes, body parts, eyeglasses, headset or the like. Determining the position of the marks and tags may provide a viewing direction. For example, the operator may wear a tag on a headset or eyeglasses; the tag may have a particular orientation (e.g. may consist of an arrow, attached to the headset, pointing towards a viewing direction); a viewing direction may be found by determining the tag's position and tag's orientation using a pattern recognition software or the like. In another exemplary embodiment, the operator may have one or more small paint patches painted on one or more parts of the body (e.g. forehead, nose tip, fingers etc.); the paint may have a specific color or fluorescence that the ultrasound system may recognize; the disposition and configuration of the various paint patches on operator's bods may provide a viewing direction of the operator. The patches of paint may be used to identify various body parts of the operator.

In another exemplary embodiment, similar to the method of identifying the probe 290, the ultrasound diagnostic device 210 may determine operator's position and viewing direction by using an infrared ray that is emitted into a predetermined space and detecting a reflected or absorbed infrared ray. An infrared reflection portion for reflecting an infrared ray or an infrared absorption portion for absorbing an infrared ray may be attached on the operator. Also, the ultrasound diagnostic device 210 may determine operator's position or viewing direction by using short range communication such as RFID, Bluetooth, etc.

The methods and procedures described in this application may be combined with each other in various ways such as to obtain various methods for determining operator's viewing position and the positions of the other objects in the room. For example, the methods described in FIG. 7A may be used in conjunction with the infrared detection sensor and the imaging of the marks and tags attached to the operator. This way, various combinations of methods, procedures, and devices may be used function of the desired parameters of the ultrasound diagnostic device such as cost of the ultrasound system, performance, ease of use, room configuration, economic feasibility etc. Further, the above methods and devices may be combined with each other and with other methods in various ways in order to custom design the desired method for determining the viewing direction of the operator.

FIG. 10 is a flowchart for explaining Operation S330 of FIG. 3 in detail. In Operation S1010, the ultrasound diagnostic device 210 determines a second position to which the display 250 is to be moved in consideration of a positional relationship between the ultrasound diagnostic device 210 and an operator. Since an area in which the display 250 connected to the ultrasound diagnostic device 210 may move is limited, the second position to which the display 250 is to be moved is determined within the area where the display 250 may move.

In Operation S1020, the ultrasound diagnostic device 210 determines a movement path from the first position where the display 250 is located to the second position. In detail, the ultrasound diagnostic device 210 may determine a movement path to guide the display 250 not to collide against a person or an object while moving from the first position to the second position.

In Operation S1030, the ultrasound diagnostic device 210 changes the position of the display 250 according to the determined movement path.

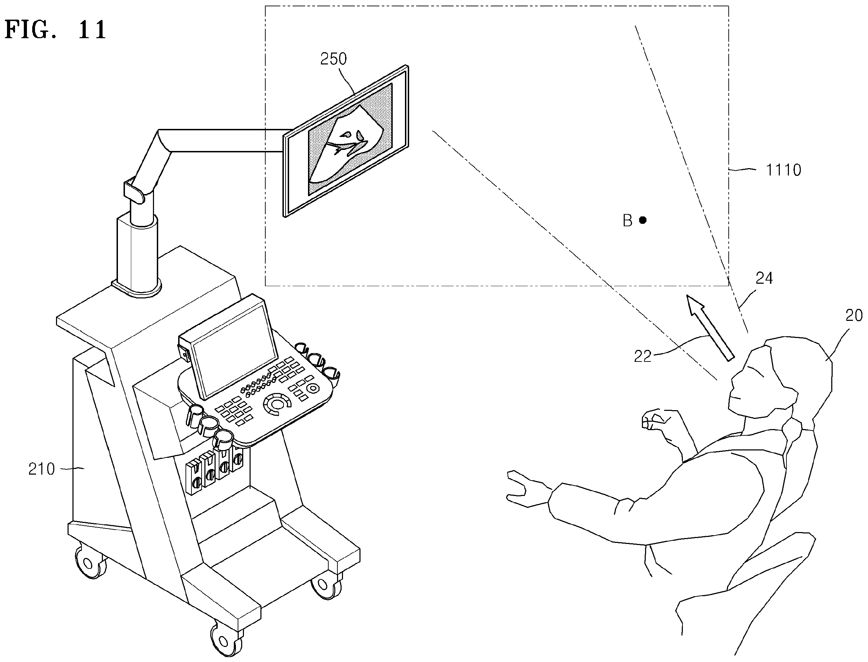

FIG. 11 illustrates a method of determining the second position considering a positional relationship between the ultrasound diagnostic device ultrasound diagnostic device 210 and the operator 20. First, the ultrasound diagnostic device 210 may determine the visible range 24 of the operator 20 based on the position and the viewing direction 22 of the operator 20. The visible range 24 of the operator 20 does not simply signify a direction in which the eyes of the operator 20 face but signifies a range in which a field of vision of the operator 20 is secured with respect to the direction in which the eyes of the operator 20 face. The visible range 24 may be determined by using statistical information about a viewing angle of an ordinary person or in various ways by the operator 20. Thus, after determining the viewing range of the operator, the ultrasound diagnostic device 210 may determine whether the operator looks at the display or whether the operator looks at other objects in the room such as the probe, the investigated person or object, or in other directions.

As illustrated in FIG. 11, a position B that is located in the visible range 24 of the operator 20 in an area 1110 within which the display 250 of the ultrasound diagnostic device 210 may move may be determined to be the second position. When there is no position that is located in the visible range 24 of the operator 20 in the area 1110 within which the display 250 may move, in other words, there is no position commonly included in the visible range 24 of the operator 20 and the area 1110 within which the display 250 may move, a position located within a predetermined distance from the visible range 24 of the operator 20 in the area 1110 within which the display 250 may move may be determined to be the second position. Thus, the display may be kept still when the operator does not look at the display.

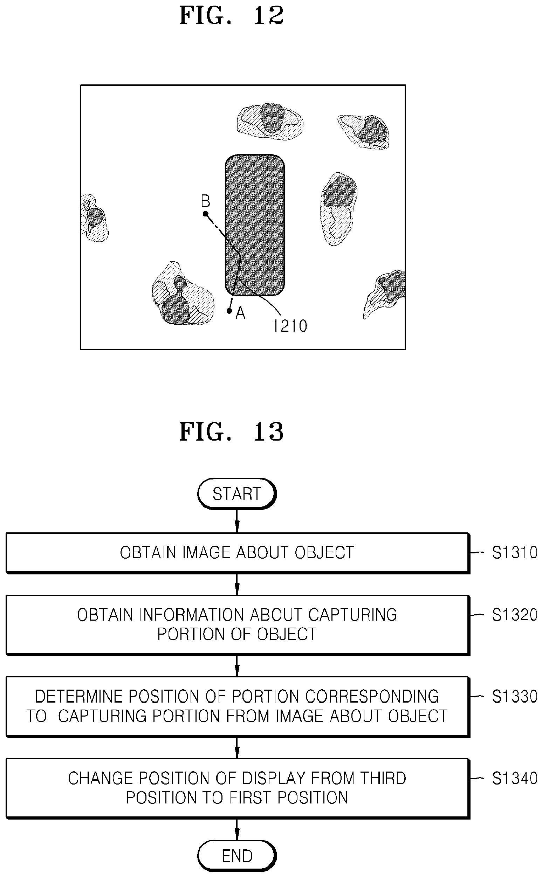

FIG. 12 illustrates a method of determining a movement path 1210 between the first position A and the second position B from an image of an operator. FIG. 12 illustrates a depth image of an operator.

When the first position A and the second position B are determined, the ultrasound diagnostic device 210 according to the present embodiment, referring to a depth image, may determined the movement path 1210 that guides the display 250 not to collide against other persons or objects while moving from the first position A to the second position B.

Since the depth image includes information about distances of the objects or persons included in the depth image, the ultrasound diagnostic device 210 prevents the display 250 from colliding against the objects or persons considering the height and width of the display 250.

The ultrasound diagnostic device 210 may change the movement path 1210 of the display 250 when an obstacle is determined to exist on the movement path 1210 of the display 250 while the display 250 is actually moved along the movement path 1210. This is because the position of an object or a person included in the depth image may be changed during the movement of the display 250.

Weather an obstacle exists on the movement path 1210 of the display 250 may be determined from the depth image or by a distance detection sensor attached on the display 250.

FIG. 13 is a flowchart for explaining a method of moving the display 250 of the ultrasound diagnostic device 210, according to another exemplary embodiment. The method of moving the display 250 of the ultrasound diagnostic device 210 according to FIG. 13 relates to a method of moving the display 250 in advance by using information about a portion of an object before the movement of the display 250 according to the position and the viewing direction of an operator.

In Operation S1310, the ultrasound diagnostic device 210 obtains an image of an object. The ultrasound diagnostic device 210 may receive the image of an object from an external device or server or by using a color camera, a black and white camera, or a depth camera. The image of an object may include a depth image of the object.

In Operation S1320, the ultrasound diagnostic device 210 obtains information about the portion of the object. For example, the ultrasound diagnostic device 210 may obtain information about which part of the object may be captured. The portion may include a head, a neck, an abdomen, a foot, etc. The ultrasound diagnostic device 210 may obtain the information about the portion of an object from an external server through a wired and/or wireless network.

In Operation S1330, the ultrasound diagnostic device 210 determines the position of a portion corresponding to the portion of an object, referring to an image of the object. For example, when the portion of an object is an abdomen, the ultrasound diagnostic device 210 identifies an abdomen portion of the object by applying an image processing method such as pattern matching to an image of the object and determines the position of an identified abdomen portion.

In Operation S1340, the ultrasound diagnostic device 210 changes the position of the display 250 from the third position to the first position.

According to the method of moving the display 250 of the ultrasound diagnostic device 210 according to the present embodiment, the display 250 may be moved in advance based on information about a portion of an object before a viewing direction of an operator is determined. Accordingly, when the display 250 is moved again according to the viewing direction of the operator, a movement time of the display 250 may be reduced.

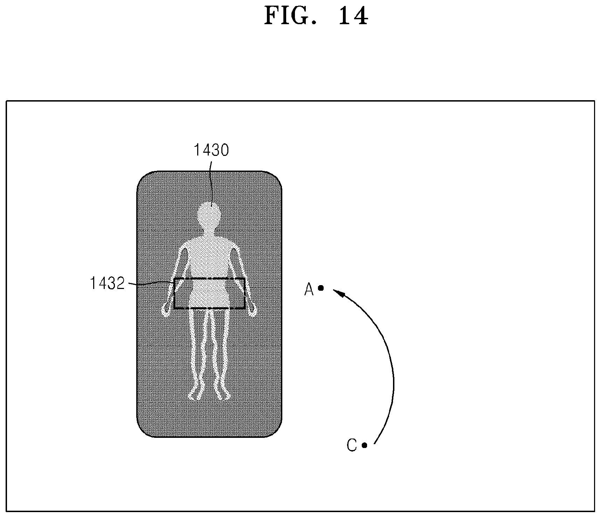

FIG. 14 illustrates a method of moving the display 250 from the third position C to the first position A. FIG. 14 illustrates a depth image of an object 1430.

When a portion of the object 1430 is an abdomen, the ultrasound diagnostic device 210 identifies an abdomen portion 1432 of the object 1430 in a depth image. Next, the ultrasound diagnostic device 210 moves the display 250 located at the third position C to the first position A close to the abdomen portion 1432 of the object.

The first position a may be determined considering an average basic posture of an operator capturing the abdomen portion 1432 of the object. For example, an average basic posture of an operator may be determined from the postures of operators capturing the abdomen portion 1432 of the object and then the first position A may be determined according to the position and viewing direction of the operator when the operator takes the average basic posture.

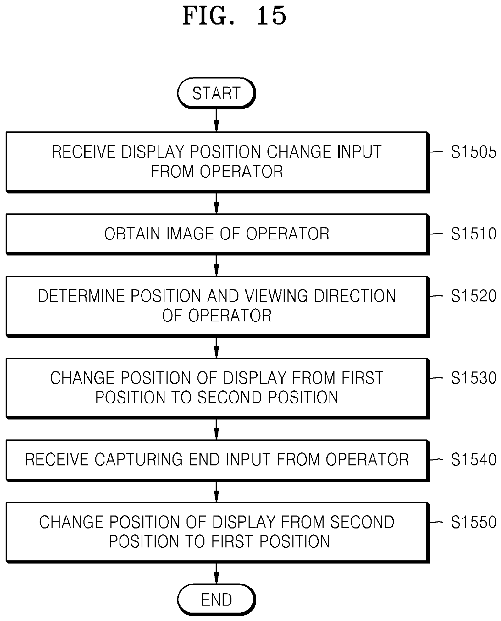

FIG. 15 is a flowchart for explaining a method of moving the display 250 of the ultrasound diagnostic device 210, according to another exemplary embodiment. Referring to FIG. 15, in Operation S1505, the ultrasound diagnostic device 210 receives an operator's input of a change of the position of the display 250. The operator may input a position change input to the ultrasound diagnostic device 210 by using at least one of a foot switch, voice, and gesture.

When the ultrasound diagnostic device 210 is located in an operation room, the operator may directly change the position of the display 250, or input a position change input, by using a hand, an object may be infected by germs because the operator's hand may be infected by the germs. Accordingly, when the operator inputs a position change by using at least one of a foot switch, voice, and gesture, the operator may be prevented from being infected by germs. The position change of the display 250 may be input by using a touch screen, a track ball, a button, etc.

In Operation S1510, the ultrasound diagnostic device 210 obtains an image of the operator. In Operation S1520, the ultrasound diagnostic device 210 determines the position and viewing direction of the operator by using an image of the operator. In Operation S1530, the ultrasound diagnostic device 210 changes the position of the display 250 from a first position to a second position according to the position and viewing direction of the operator.

In Operation 1540, the ultrasound diagnostic device 210 receives a capturing end input command from the operator. The capturing end input command may signal to the ultrasound diagnostic device that the recording or capturing of ultrasound diagnostic image data has been completed. Like the position change input, the capturing end input may include at least one of an input through a foot switch, an input through operator's voice, and an input through operator's gesture.

In Operation S1550, the ultrasound diagnostic device 210 changes the position of the display 250 from the second position to the first position according to the image capturing end input. This is to change the position of the display 250 to the original position after the image capturing ends. The ultrasound diagnostic device 210 may determine a movement path to move the display 250 from the second position to the first position from an image of the operator. When an obstacle is determined to exist on the movement path during the movement of the display 250, the ultrasound diagnostic device 210 may change the movement path of the display 250.

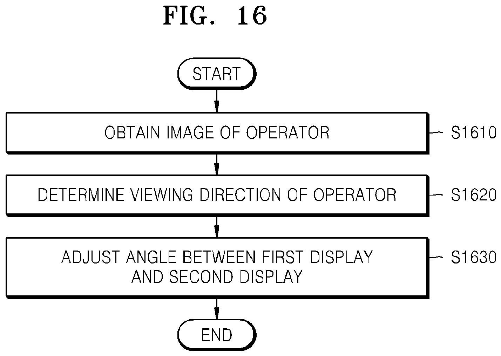

FIG. 16 is a flowchart for explaining a method of moving a display of the ultrasound diagnostic device 210, according to another exemplary embodiment. In FIG. 16, the ultrasound diagnostic device 210 may include a first display and a second display connected to each other.

In Operation S1610, the ultrasound diagnostic device 210 obtains an image of an operator. In Operation S1620, the ultrasound diagnostic device 210 determines a viewing direction of the operator from an image of the operator. Since a method of determining a viewing direction of the operator by using an image of the operator is already described above, a detailed description thereof will be omitted herein.

In Operation 1630, the ultrasound diagnostic device 210 adjusts an angle between the first and second displays according to a viewing direction of the operator. The angle between the first and second displays may be variously set by the operator.

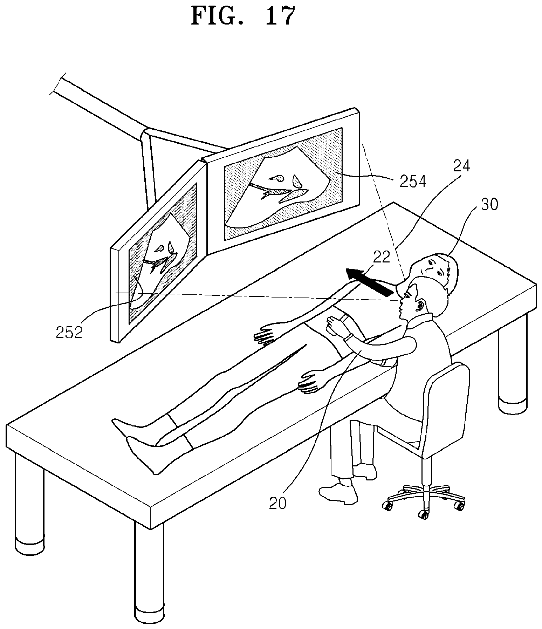

FIG. 17 illustrates a method of adjusting an angle between a first display 252 and a second display 254. Referring to FIG. 17, the ultrasound diagnostic device 210 may adjust the angle between the first display 252 and the second display 254 according to the viewing direction 22 of the operator 20. Accordingly, a screen of the first display 252 and a screen of the second display 254 may be located in the visible range 24 of the operator 20. Although FIG. 17 illustrates two displays 252 and 254 only, a person of ordinary skill in the art would understand that more than two displays may be included in the ultrasound diagnostic device.

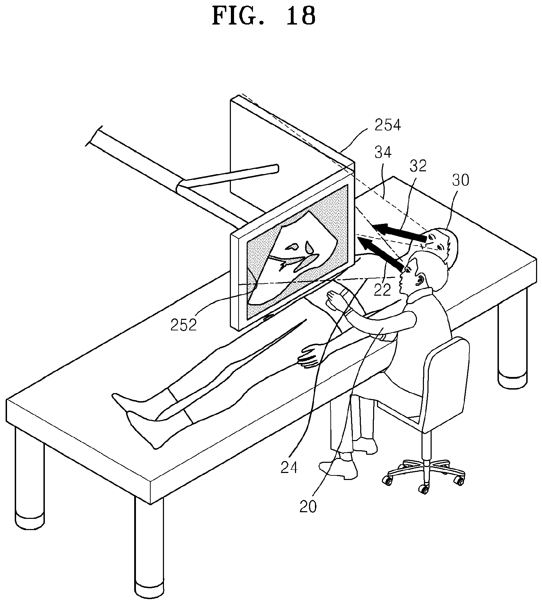

FIG. 18 illustrates another method of adjusting an angle between the first display 252 and the second display 254. Referring to FIG. 18, the ultrasound diagnostic device 210 may determine the viewing direction 22 of the operator 20 from an image of the operator 20 and a viewing direction 32 of the object 30 from an image of the object 30. The object 30 may be a person. The ultrasound diagnostic device 210 may adjust an angle between the first display 252 and the second display 254 according to the viewing direction 22 of the user 20 and the viewing direction 32 of the object 30. Accordingly, the screen of the first display 252 may be located within the viewing range 24 of the operator 20 and the screen of the second display 254 may be located within the viewing range 34 of the object 30.

In the ultrasound diagnostic device 210 used to observe a fetus, the object 30 often wants to directly see a fetus. Thus, in the ultrasound diagnostic device 210 according to the present embodiment, the operator 20 may see the first display 252 and the object 30 may see the second display 254. In other words, referring to FIGS. 17 and 18, the ultrasound diagnostic device 210 according to the present embodiment may include two displays and the operator 20 may use both of the two displays or one of the two displays may be used by the object 30.

Conventionally, a display for an object and a display for an operator are separately connected to an ultrasound diagnostic device. In the ultrasound diagnostic device 210 according to the present embodiment, without distinguishing a display for an object and a display for an operator, two displays may be use for a variety of purposes by adjusting an angle between the two displays for convenience sake. Also, when there is a preset value by the operator 20 about the angle between two displays, the angle between the two displays may be adjusted according to the preset value.

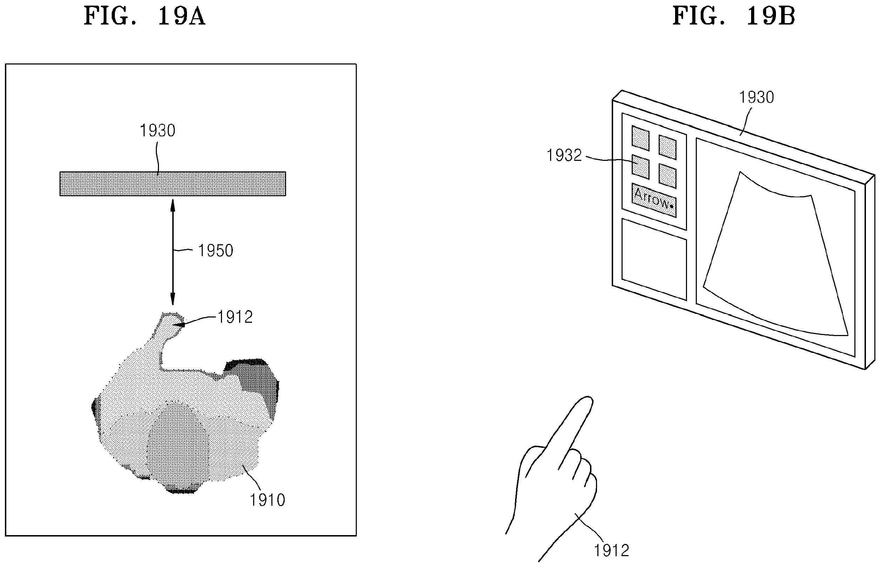

The ultrasound diagnostic device 210 according to the present embodiment may detect operator's gesture regarding a display located at the second position and perform a function of the ultrasound diagnostic device 210 corresponding to the operator's gesture. As described above, when the ultrasound diagnostic device 210 is located in an operation room, it is very important to recognize the user's gesture in order to prevent the operator's hands from being infected by germs, which will be described with reference to FIG. 19.

FIG. 19A illustrates a method of detecting gesture of an operator 1910 from a depth image. FIG. 19B illustrates a method of performing a function of the ultrasound diagnostic device 210 displayed on a display 1930 according to gesture of the operator 1910.

FIG. 19A illustrates a depth image including the user 1910 and the display 1930 located at the second position. When it is determined that a hand 1912 or a finger of the operator 1910 and the display 1930 included in the depth image are located for a predetermined time within a preset distance 1950, the ultrasound diagnostic device 210 may detect that a gesture motion by the operator 1910 is input.

The operator may have one or more identifying patches attached or painted on his hand or fingers. The patches may have specific colors, fluorescence, or shapes that the ultrasound diagnostic device may identify by using an image an color recognition software. The patches may be used to identify specific fingers or parts of the hand of the operator. Different colors and fluorescent degrees may be used to tag different fingers. Further, the above methods and devices may be combined with each other and with other methods in various ways in order to custom design the desired method for determining the gestures of the operator.

Referring to FIG. 19B, when detecting that a gesture motion is input by the operator 1910, the ultrasound diagnostic device 210 may perform a function 1932 indicated by the hand 1912 of the operator 1910 among functions of the ultrasound diagnostic device 210 displayed on the display 1930.

Also, the ultrasound diagnostic device 210 may detect the gesture motion of the operator 1910 by using a gesture detection camera attached on the display 1930. Since a method of detecting a gesture motion of the operator 1910 through an image of an operator captured by a gesture detection camera is a well-known technology to one of ordinary person in the art, a detailed description thereof will be omitted herein.

The ultrasound diagnostic device 210 may detect gesture of eyes of the operator 1910 by using an eye tracking sensor attached on the display 1930 in addition to the depth image. In detail, the eye tracking sensor may track a direction indicated by an eyeball of the operator 1910 by detecting a movement of the eyeball of the operator 1910. Then, the eye tracking sensor may identify an eye blinking gesture input, a voice input, and a foot input of the operator 1910 and perform a function corresponding to the direction indicated by an eyeball of the operator 1910 among the function displayed on the display 1930.

The ultrasound diagnostic device 210 may manually change the position of the display 1930 according to the gesture motion of the operator 1910. The operator 1910 may finely adjust the position of the display 1930 located at the second position through the gesture motion.

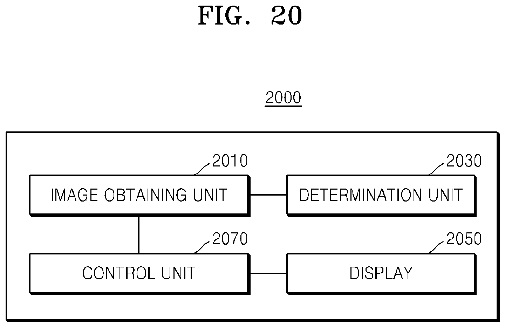

FIG. 20 is a block diagram illustrating the structure of an ultrasound diagnostic device 2000 according to an exemplary embodiment. Referring to FIG. 20, the ultrasound diagnostic device 2000 according to the present embodiment may include an image obtaining unit 2010, a determination unit 2030, a display 2050, and a control unit 2070. The image obtaining unit 2010, the determination unit 2030, and the control unit 2070 may be embodied by a microprocessor.

The image obtaining unit 2010 may receive an image of an operator or an object from an external device or obtain the image by using a color camera, a black and white camera, or a depth camera. The image of an operator or an object may include a depth image and may be an image separately captured for each of an operator and an object or an image including both of the operator and the object.

The determination unit 2030 may determine the position and viewing direction of an operator based on the image of the operator. Also, the determination unit 2030 may determine a visible range of an operator from the position and viewing direction of the operator. Since a method of determining a visible range of an operator from the image of the operator is already described above, a detailed description thereof will be omitted herein.

The display 2050 displays at least one of information about an object and an ultrasound image of the object on a screen. The information about an object may include name, age, portion, etc. of the object. The ultrasound image of an object may include a B-mode image, an M-mode image, a Doppler image, etc.

The display 2050 may include a CRT, an LCD, a PDP, an OLED, an FED, an LED, a VFD, a DLP, a PFD, a 3D display, a transparent display, etc. and may include a variety of display within a range that is obvious to one of ordinary skill in the art.

The control unit 2070 may change the position of the display 2050 from the first position to the second position according to the position and viewing direction of an operator. The control unit 2070 may determine the second position according to a relative positional relationship between the ultrasound diagnostic device 2000 and the operator before the position of the display 2050 is changed and determine a movement path from the first position to the second position referring to an image of an operator. Also, when an obstacle is determined to exist on a movement path of the display 2050 by using a distance detection sensor attached on the display 2050, the control unit 2070 changes the movement path of the display 2050 so that collision against the obstacle may be prevented.

The display 2050 of the ultrasound diagnostic device 2000 according to the present embodiment may include a first display and a second display that display at least one of information about an object and an ultrasound image of the object.

The control unit 2070 may adjust an angle between the first display and the second display. In detail, the control unit 2070 may adjust the angle between the first display and the second display according to the viewing direction of the operator such that a screen of the first display and a screen of the second display are located within a visible range of the operator, or may adjust the angle between the first display and the second display according to the viewing direction of the operator and the viewing direction of the object such that the screen of the first display is located within a visible range of the operator and the screen of the second display is located within a visible range of the object.

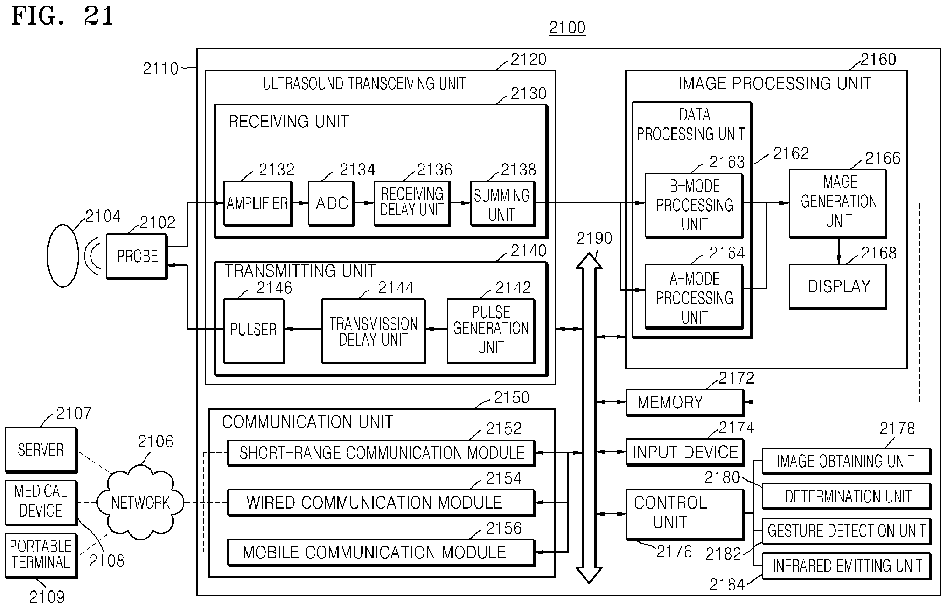

FIG. 21 is a block diagram illustrating the structure of an ultrasound diagnostic device 2100 according to another exemplary embodiment. Referring to FIG. 21, the ultrasound diagnostic device 2100 may include a probe 2102, an ultrasound transceiving unit 2120, an image processing unit 2160, a communication unit 2150, a memory 2172, an input device 2174, a control unit 2176, an image obtaining unit 2178, a determination unit 2180, a gesture detection unit 2182, and an infrared emitting unit 2184. The above-described elements may be connected to each other via a bus 2190.

The ultrasound diagnostic device 2100 may be embodied in not only a cart type or a portable type. A portably ultrasound diagnostic device may be, for example, a PACS viewer, a smartphone, a laptop computer, a PDA, a tablet PC, etc., but the exemplary embodiments are not limited thereto.

The probe 2102 transmits an ultrasound signal to an object 2104 according to a driving signal applied by the ultrasound transceiving unit 2120 and receives a response signal reflected from the object 2104. The probe 2102 includes a plurality of transducers. Each transducer generates an ultrasound wave that is acoustic energy and vibrates according to a transferred electrical signal. Also, the probe 2102 may be connected with a main body of the ultrasound diagnostic device 2100 in a wired or wireless way. The ultrasound diagnostic device 2100 may be provided with a plurality of the probes 2102 according to an embodiment type.

The transmission unit 2140 supplies a driving signal to the probe 2102 and includes a pulse generation unit 2142, a transmission delay unit 2144, and a pulser 2146. The pulse generation unit 2142 generates pulses for forming a transmission ultrasound wave according to a predetermined pulse repetition frequency (PRF). The transmission delay unit 2144 applies a delay time to determine transmission directionality to a pulse. Each pulse to which a delay time is applied corresponds to each of a plurality of piezoelectric vibrators included in the probe 2102. The pulse 2146 applies a driving signal or a driving pulse to the probe 2102 at a timing corresponding to each pulse to which the delay time is applied.

The receiving unit 2130 may generate ultrasound data by processing a response signal received from the probe 2102 and may include an amplifier 2132, an analog-to-digital converter (ADC) 2134, a receiving delay unit 2136, and a summing unit 2138. The amplifier 2132 amplifies a response signal for each channel. The ADC 2134 performs analog-to-digital conversion on an amplified response signal. The receiving delay unit 2136 applies a delay time to determine reception directionality to a digitally converted response signal. The summing unit 2138 generates ultrasound data by summing the response signal processed by the receiving delay unit 2136.

The image processing unit 2160 generates and displays an ultrasound image through a scan conversion process on the ultrasound data generated by the ultrasound transceiving unit 2120. An ultrasound image may be presented not only as a gray scale ultrasound image obtained by scanning an object according to an amplitude (A) mode, a brightness (B) mode, and a motion (M) mode, but also as a Doppler image representing a motion of an object. A Doppler image may include a blood flow Doppler image (referred to as a color Doppler image) indicating the flow of blood, a tissue Doppler image indicating a motion of tissue, and a spectral Doppler image displaying a movement speed of an object as a waveform.

A B-mode processing unit 2163 extracts a B-mode component from ultrasound data and processes the extracted component. An image generation unit 2166 may generate an ultrasound image, in which signal strength is presented by brightness, based on the B-mode component extracted by the B-mode processing unit 2163.

Likewise, the Doppler processing unit 2164 extracts a Doppler component from the ultrasound data and the image generation unit 2166 may generate a Doppler image in which a movement of an object is presented by a color or a waveform based on the extracted Doppler component.

The image generation unit 2166 according to the present embodiment may generate a 3D ultrasound image through a volume rendering process on volume data and generate an elastic image obtained by visualizing a degree of deformation of the object 2104 according to pressure. Furthermore, the image generation unit 2166 may express various pieces of additional information by text or graphics on the ultrasound image. The generated ultrasound image may be stored in the memory 2172.

The display 2168 displays the generated ultrasound image. The display 2168 may display not only an ultrasound image but also various pieces of information processed by the ultrasound diagnostic device 2100 on a screen through a graphic user interface (GUI). The ultrasound diagnostic device 2100 may include two more displays 2168 according to an embodiment type. Also, as described above, the display 2168 may be connected to the ultrasound diagnostic device 2100 via an arm having a degree of freedom of 6 axes and may be moved to a certain position by the arm. However, aspects of the exemplary embodiments are not limited thereto, such that other systems for moving the display, which may have other number of degrees of freedom, may be used.

The communication unit 2150 is connected to a network 2106 in a wired or wireless way to communicate with an external device or server. The communication unit 2150 may communicate data with a server or other medical device in a hospital via a picture archiving and communication system (PACS). Also, the communication unit 2150 may communicate data according to a digital imaging and communications in medicine (DICOM).

The communication unit 2150 may transceive data related to diagnosis of an object such as an ultrasound image, ultrasound data, Doppler data, etc. of the object through the network 2106 and also transceive a medical image captured by other medical device such as a CT, an MRI, an X-ray, etc. Furthermore, the communication unit 2150 may receive information about a diagnosis history or treatment schedule of a patient from a server and use the received information for diagnosis of the object. The communication unit 2150 may perform data communication not only with a server or medical devices in a hospital but also with a portable terminal of a medical doctor or a patient.

The communication unit 2150 may perform communication with an external device or server and obtain information about a portion of an object. The determination unit 2180 may determine the position of a portion corresponding to the portion of an object from an image of the object and change the position of the display 2168 from the third position to the first position according to the position of the determined portion. Accordingly, the 2100 may move in advance the display 2158 according to information about the portion of the object so as to reduce a movement time to move the display 2168 along the viewing direction of the operator.

The communication unit 2150 may receive a position change input of the display 2168 by using at least one of a foot switch, voice, and gesture, by the operator. In this case, the control unit 2176 may determine the viewing direction of the operator according to the operator's position change input and then change the position of the display 2168 from the first position to the second position. Also, the control unit 2176 may change the position of the display 2168 from the third position to the first position based on information about the portion of the object according to the operator's position change input. The communication unit 2150 may received a capturing end input by using at least one of a foot switch, voice, and gesture by the operator. The control unit 2176 may change the position of the display 2168 from the first position to the second or third position according to the capturing end input.

The communication unit 2150 is connected to the network 2106 in a wired or wireless way to communicate data with a server 2107, a medical device 2108, or a portable terminal 2109. The communication unit 2150 may include one or more constituent elements that enable communication with an external device, for example, a short-range communication module 2152, a wired communication module 2154, and a mobile communication module 2156.

The short-range communication module 2152 signifies a module for short-range communication within a predetermined distance. The short-range communication technology includes, for example, wireless LAN, Wi-Fi, Bluetooth, Zigbee, Wi-Fi Direct (WFD), ultra wideband (UWB), infrared data association (IrDA), Bluetooth low energy (BLE), near field communication (NFC), etc., but the exemplary embodiments are not limited thereto.

The wired communication module 2154 signifies a module for communication using an electrical signal or an optical signal. A wired communication technology according to an embodiment may include a pair cable, a coaxial case, an optical fiber cable, an Ethernet cable, etc.

The mobile communication module 2156 transceives a wireless signal with at least one of a local station, an external terminal, and a server on a mobile communication network. The wireless signal may include a variety of forms of data according to communication of a voice call signal, a video call signal, or a text and/or multimedia message.

The memory 2172 stores various pieces of information processed by the ultrasound diagnostic device 2100. For example, the memory 2172 may store medical data related to diagnosis of an object such as ultrasound data, an ultrasound image, etc. that is input or output, or an algorithm or a program executed in the ultrasound diagnostic device 2100.

The memory 2172 may be embodied by a variety of types of storage media such as flash memory, a hard disk, EEPROM, etc. Also, the ultrasound diagnostic device 2100 may employ a web storage or a cloud server performing a storage function of the memory 2172 on a web.

The input device 2174 signifies a unit for receiving an input of data from an operator to control the ultrasound diagnostic device 2100. The input device 2174 may include hardware such as a keypad, a mouse, a touch panel, a touch screen, a trackball, a jog switch, etc., but the exemplary embodiments are not limited thereto and the input device 2174 may further include various input units such as an electrocardiogram measuring module, a respiration measuring module, a voice recognition sensor, a gesture recognition sensor, a fingerprint recognition sensor, an iris recognition sensor, a depth sensor, a distance sensor, etc.

The control unit 2176 controls the overall operation of the ultrasound diagnostic device 2100. In other words, the control unit 2176 may control the operations among the probe 2102, the ultrasound transceiving unit 2120, the image processing unit 2160, the communication unit 2150, the memory 2172, the input device 2174, the image obtaining unit 2178, the determination unit 2180, the gesture detection unit 2182, and the infrared emitting unit 2184. The control unit 2176 may change the position of the display 2168 by controlling the arm to which the display 2168 is connected.

The image obtaining unit 2178 may receive an image of an operator or the object 2104 from an external device or an external server 2107 and obtain the image of an operator or the object 2104 by using a color camera, a black and white camera, or a depth camera.

The determination unit 2180 may determine the position, viewing direction, and visible range of an operator or the object 2104 based on the image of an operator or the object 2104.

The gesture detection unit 2182 may detect gestures of an operator with respect to the display 2168 located at the second position. The control unit 2176 may perform a function of the ultrasound diagnostic device 2100 corresponding to the gesture motion detected by the gesture detection unit 2182. The gesture detection unit 2182 may detect gesture of an operator by using the image of an operator, a gesture detection camera attached on the display 2168, or an eye tracking sensor attached on the display 2168.

The infrared emitting unit 2184 emits an infrared ray into a predetermined space including an operator or the ultrasound diagnostic device 2100. The control unit 2176 may determine the position of an object that reflects or absorbs the emitted infrared ray as the position of the probe 2102 and determine as the operator a person who is the closest to the position of the probe 2102 among a plurality of persons included in the image of an operator. When the image obtaining unit 2178 of the ultrasound diagnostic device 2100 according to the present embodiment includes a depth camera that uses an infrared method, the position of the probe 2102 may be determined by using an infrared ray emitted by the depth camera, instead of the infrared ray emitted by the infrared emitting unit 2184.

Part or the whole of the probe 2102, the ultrasound transceiving unit 2120, the image processing unit 2160, the communication unit 2150, the memory 2172, the input device 2174, the control unit 2176, the image obtaining unit 2178, the determination unit 2180, the gesture detection unit 2182, and the infrared emitting unit 2184 may be operated by a software module, but the exemplary embodiments are not limited thereto and part of the above-described structure may be operated by hardware. Also, at least part of the ultrasound transceiving unit 2120, the image processing unit 2160, and the communication unit 2150 may be included in the control unit 2176, but the exemplary embodiments are not limited to such an embodiment.

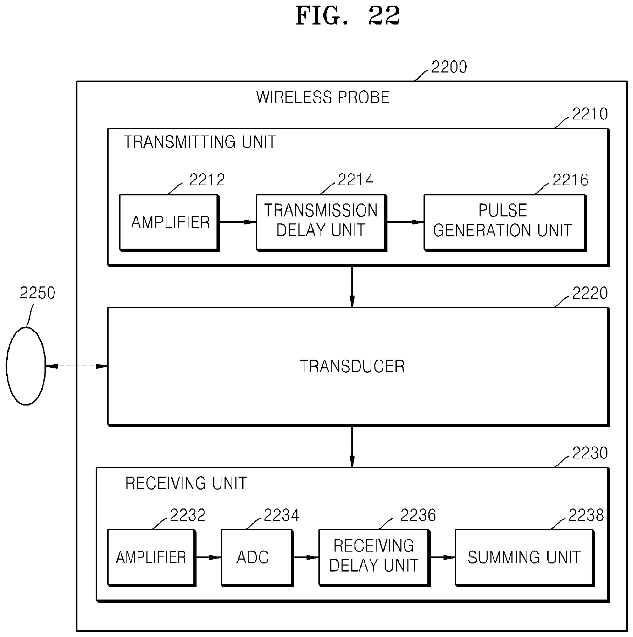

FIG. 22 is a block diagram illustrating the structure of a wireless probe 2200 that may be connected to an ultrasound diagnostic device, according to an exemplary embodiment. The wireless probe 2200 of FIG. 22 may include a plurality of transducers as described in FIG. 21 and may include part or the whole of the structure of the ultrasound transceiving unit 2120 of FIG. 21.

The wireless probe 2200 according to the present embodiment of FIG. 22 includes a transmitting unit 2210, a transducer 2220, and a receiving unit 2230, whose structures are already described with reference to FIG. 21 and thus detailed descriptions thereof will be omitted herein. The wireless probe 2200 may selectively include a receiving delay unit 2236 and a summing unit 2238 according to an embodiment type thereof.

The wireless probe 2200 may transmit an ultrasound signal to an object 2250 and receive a response signal therefrom, or generate ultrasound data and wirelessly transmit the generated ultrasound data to the ultrasound diagnostic device 2100 of FIG. 21.

The exemplary embodiments can also be embodied as computer readable codes on a computer readable recording medium. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, optical data storage devices, etc. The computer readable recording medium can also be distributed over network coupled computer systems so that the computer readable code is stored and executed in a distributed fashion.

While this invention has been particularly shown and described with reference to exemplary embodiments thereof, it will be understood by those skilled in the art that various changes in form and details may be made therein without departing from the scope of the invention as defined by the appended claims.

In addition, many modifications can be made to adapt a particular situation or material to the teachings of the present disclosure without departing from the essential scope thereof. Therefore, it is intended that the present disclosure not be limited to the particular exemplary embodiments disclosed as the best mode contemplated for carrying out the present disclosure, but that the present disclosure will include all embodiments falling within the scope of the appended claims.

## Claims

1. A method of operating a display of an ultrasound diagnostic device having at least one display, the method comprising:
obtaining an image of an object;
obtaining information about a portion of the object that will be captured with the ultrasound diagnostic device;
determining the position of the portion of the object from the image of the object;
changing the position of the display based on the determined position of the portion of the object;
obtaining an image of an operator of the ultrasound diagnostic device performing ultrasound imaging of the object by using the ultrasound diagnostic device;
determining at least a viewing direction and optionally a position of the operator from the obtained image of the operator; and
changing at least one of
an orientation from a first angle to a second angle, and
a position from a first position to a second position of the display connected to the ultrasound diagnostic device according to at least the determined viewing direction and optionally the determined position of the operator.

2. The method of claim 1, wherein the changing of the position of the display comprises:
determining a viewing range of the operator based on the determined viewing direction and optionally the determined position of the operator; and
changing the orientation and/or the position of the display, wherein a screen of the display is located in the determined viewing range of the operator,
wherein optionally the changing of the position of the display comprises determining a position located in the viewing range of the operator and in a space within which the display is movable as the second position.

3. The method of claim 1 or 2, wherein the determining of the viewing direction of the operator comprises:
determining a posture of the operator based on the image of the operator; and
determining the viewing direction of the operator by using the determined posture of the operator,
wherein optionally further the determining of the posture of the operator comprises determining the posture of the operator based on at least one of a shape and a positional relationship of a head part and a body part of the operator included in the image of the operator.

4. The method of claim 1, 2 or 3, wherein the image of the operator further comprises a support table supporting the object, and
the determining of at least the viewing direction and/or the determining of the position of the operator comprises determining a positional relationship between the operator and the support table comprised in the image of the operator.

5. The method of any one or more than one of the preceding claims, wherein the changing of the position of the display comprises determining the second position, and the determining of the second position comprises determining a positional relationship between the ultrasound diagnostic device and the operator.

6. The method of any one or more than one of the preceding claims, wherein:
the obtaining of the image of the operator comprises obtaining a depth image of the operator, and
the determining of at least the viewing direction and optionally the position of the operator comprises determining the viewing direction and optionally the position of the operator based on the depth image of the operator,
wherein the obtaining of the depth image comprises any one or more than one of:
obtaining a depth image of the operator by using a depth camera; and
obtaining images of the operator by using at least two color cameras and obtaining a depth image of the operator by applying stereo matching to the images of the operator obtained by using the at least two color cameras.

7. The method of any one or more than one of the preceding claims, wherein the changing of the position of the display comprises determining a movement path from a first angle to a second angle and/or from the first position to the second position based on information about a position of at least one of a person and an object comprised in the image of the operator, and
wherein optionally the determining of the movement path comprises determining the movement path from a first angle to a second angle and/or from the first position to the second position such that the display does not to collide with at least one of an obstacle, the person and the object included in the image of the operator.

8. The method of any one or more than one of the preceding claims, wherein the changing of the position of the display comprises changing from a first movement path to a second movement path of the display upon determining that an obstacle exists on the first movement path of the display;
wherein no obstacle exists on the second movement path; and
wherein the first and second movement paths are moving paths between the first position of the display and the second position of the display.

9. The method of any one or more than one of the preceding claims, wherein the determining of at least the viewing direction and optionally of the position of the operator comprises determining a person as the operator, which person is the closest to a position of a probe of the ultrasound diagnostic device when a plurality of persons are comprised in the image of the operator,
wherein optionally the determining of the person who is the closest to the position of the probe of the ultrasound diagnostic device as the operator comprises:
determining the position of the probe by using infrared or short-range communication; and
determining a person who is the closest to the position of the probe, based on the position of the probe and the image of the operator, as the operator.

10. The method of any one or more than one of the preceding claims, wherein the display comprises a first display and a second display, and
the method further comprising adjusting an angle between the first display and the second display,
wherein optionally the adjusting of the angle between the first display and the second display comprises either:
adjusting the angle between the first display and the second display such that a screen of the first display and a screen of the second display are located at the viewing direction of the operator; or
adjusting the angle between the first display and the second display so that a screen of the first display is located at the viewing direction of the operator and the screen of the second display is located at a viewing direction of the object.

11. The method of any one or more than one of the preceding claims, further comprising:
detecting a gesture of the operator with respect to the display located at the second position; and
performing a function of the ultrasound diagnostic device corresponding to the gesture of the operator,
wherein the detecting of the gesture of the operator optionally comprises:
detecting a gesture of the operator by using the image of the operator, and wherein the detecting of the gesture of the operator optionally comprises performing a function corresponding to a position indicated by a hand of the operator among functions displayed on the display when it is determined, based on the image of the operator, that the hand of the operator and the display are located within a preset distance for a preset time, or
detecting a gesture of the operator by using a gesture detection camera attached to the display, or
detecting a gesture or movement of an eye of the operator by using an eye tracking sensor attached to the display or attached to a headset of the operator; and performing a function of the ultrasound diagnostic device corresponding to the detected gesture or movement of the eye of the operator.

12. The method of any one or more than one of the preceding claims, further comprising receiving a display position change input from the operator, and the display position change input comprises at least one of an input through a foot switch connected to the ultrasound diagnostic device, an input through operator's voice, and an input through operator's gesture.

13. The method of any one or more than one of the preceding claims, further comprising:
receiving a capturing end input by the operator or receiving input from the operator to end image capturing; and
changing the position of the display from the second position to the first position according to the capturing end input.

14. A non-transitory computer readable storage medium having recorded thereon a program for executing the method according to claim 1.

15. An ultrasound diagnostic device comprising:
an image obtaining unit for obtaining an image of an operator of the ultrasound diagnostic device performing ultrasound imaging of an object by using the ultrasound diagnostic device;
a determination unit for determining at least a viewing direction and optionally a position of the operator from the obtained image of the operator;
a display for displaying at least one of an information of the object and an ultrasound image of the object; and
a control unit for changing at least one of
an orientation from a first angle to a second angle, and
a position from a first position to a second position of the display connected to the ultrasound diagnostic device according to at least the determined viewing direction and optionally the determined position of the operator, **CHARACTERISED IN THAT** the ultrasound diagnostic device further comprises:
means for obtaining an image of an object;
means for obtaining information about a portion of the object that will be captured;
wherein the determination unit is also determining the position of the portion of the object from the image of the object; and
wherein the control unit is also changing a position of the display from a third position to the first position based on the determined position of the portion of the object.

## Patentansprüche

1. Verfahren zum Bedienen der Anzeige einer Ultraschalldiagnosevorrichtung mit wenigstens einer Anzeige, wobei das Verfahren umfasst:
Erhalten eines Bildes eines Objektes;
Erhalten von Informationen über einen Abschnitt des Objektes, welcher von der Ultraschalldiagnosevorrichtung erfasst werden wird;
Bestimmen der Position des Abschnitts des Objekts aus dem Bild des Objekts;
Ändern der Position der Anzeige basierend auf der bestimmten Position des Abschnitts des Objekts;
Erhalten eines Bildes eines Bedieners der Ultraschalldiagnosevorrichtung, welcher eine Ultraschallbildgebung an dem Objekt mit Hilfe der Ultraschalldiagnosevorrichtung durchführt;
Bestimmen von wenigsten einer Betrachtungsrichtung und optional einer Position des Bedieners aus dem erhaltenen Bild des Bedieners; und
Ändern von wenigstens einer der folgenden:
einer Ausrichtung von einem ersten Winkel zu einem zweiten Winkel, und
einer Position von einer ersten Position zu einer zweiten Position der mit der Ultraschalldiagnosevorrichtung verbundenen Anzeige gemäß wenigstens der bestimmten Betrachtungsrichtung und optional der bestimmten Position des Bedieners.

2. Verfahren gemäß Anspruch 1, wobei das Ändern der Position der Anzeige umfasst:
Bestimmen eines Betrachtungsbereichs des Bedieners basierend auf der bestimmten Betrachtungsrichtung und optional der bestimmten Position des Bedieners; und
Ändern der Ausrichtung und/oder der Position der Anzeige, wobei ein Bildschirm der Anzeige in dem bestimmten Betrachtungsbereich des Bedieners angeordnet ist,
wobei optional das Ändern der Position der Anzeige umfasst: Bestimmen einer Position in dem Betrachtungsbereich des Bedieners und in einem Raum, in dem die Anzeige beweglich ist, als die zweite Position.

3. Verfahren gemäß Anspruch 1 oder 2, wobei das Bestimmen der Betrachtungsrichtung des Bedieners umfasst:
Bestimmen einer Haltung des Bedieners basierend auf dem Bild des Bedieners; und
Bestimmen der Betrachtungsrichtung des Bedieners durch Verwenden der bestimmten Haltung des Bedieners,
wobei optional das Bestimmen der Haltung des Bedieners weiterhin umfasst: Bestimmen der Haltung des Bedieners basierend auf einer Form und/oder einer Positionsbeziehung eines Kopfteils und eines Körperteils des Bedieners, die in dem Bild des Bedieners inbegriffen sind.

4. Verfahren gemäß Anspruch 1, 2 oder 3, wobei das Bild des Bedieners weiterhin einen Auflagetisch, auf dem das Objekt aufliegt, umfasst, und
wobei das Bestimmen von wenigstens der Betrachtungsrichtung und/oder das Bestimmen der Position des Bedieners umfassen: Bestimmen einer Positionsbeziehung zwischen dem Bediener und dem in dem Bild des Bedieners enthaltenen Auflagetisch.

5. Verfahren gemäß einem oder mehreren der vorangegangenen Ansprüche, wobei das Ändern der Position der Anzeige das Bestimmen der zweiten Position umfasst, und wobei das Bestimmen der zweiten Position das Bestimmen der Positionsbeziehung zwischen der Ultraschalldiagnosevorrichtung und dem Bediener umfasst.

6. Verfahren gemäß einem oder mehreren der vorangegangenen Ansprüche, wobei:
das Erhalten des Bildes des Bedieners das Erhalten eines Tiefenbildes des Bedieners umfasst, und
das Bestimmen von wenigstens der Betrachtungsrichtung und optional der Position des Bedieners umfassen: Bestimmen der Betrachtungsrichtung und optional der Position des Bedieners basierend auf dem Tiefenbild des Bedieners,
wobei das Erhalten des Tiefenbildes einen oder mehrere der folgenden Schritte umfasst:
Erhalten eines Tiefenbildes des Bedieners mit Hilfe einer Tiefenkamera; und
Erhalten von Bildern des Bedieners mit Hilfe von wenigstens zwei Farbkameras und Erhalten eines Tiefenbildes des Bedieners durch Anwenden eines Stereoabgleichs an den mit Hilfe der wenigstens zwei Farbkameras erhaltenen Bildern des Bedieners.

7. Verfahren gemäß einem oder mehreren der vorangegangenen Ansprüche, wobei das Ändern der Position der Anzeige umfasst: Bestimmen eines Bewegungspfades von einem ersten Winkel zu einem zweiten Winkel und/oder von der ersten Position zu der zweiten Position basierend auf Informationen über eine Position einer Person und/oder eines Objektes, die in dem Bild des Bedieners enthalten sind, und
wobei optional das Bestimmen des Bewegungspfades umfasst: Bestimmen des Bewegungspfades von einem ersten Winkel zu einem zweiten Winkel und/oder von der ersten Position zu der zweiten Position derart, dass die Anzeige nicht mit einem Hindernis, der Person und/oder dem Objekt, die in dem Bild des Bedieners enthalten sind, kollidiert.

8. Verfahren gemäß einem oder mehreren der vorangegangenen Ansprüche, wobei das Ändern der Position der Anzeige umfasst: Wechseln von einem ersten Bewegungspfad zu einem zweiten Bewegungspfad der Anzeige, wenn bestimmt wurde, dass auf dem ersten Bewegungspfad der Anzeige ein Hindernis vorhanden ist;
wobei kein Hindernis auf dem zweiten Bewegungspfad vorhanden ist; und
wobei es sich bei dem ersten und dem zweiten Bewegungspfad um Pfade handelt, die eine Bewegung zwischen der ersten Position der Anzeige und der zweiten Position der Anzeige ermöglichen.

9. Verfahren gemäß einem oder mehreren der vorangegangenen Ansprüche, wobei das Bestimmen von wenigstens der Betrachtungsrichtung und optional der Position des Bedieners umfassen:
Bestimmen einer Person als den Bediener, welche Person sich am nächsten zu einer Position einer Sonde der Ultraschalldiagnosevorrichtung befindet, wenn eine Mehrzahl von Personen in dem Bild des Bedieners umfasst sind,
wobei optional das Bestimmen der Person, die sich am nächsten zu der Position der Sonde der Ultraschalldiagnosevorrichtung befindet, als den Bediener, umfasst:
Bestimmen der Position der Sonde mit Hilfe von Infrarotstrahlen oder Nahbereichskommunikation; und
Bestimmen einer Person als den Bediener, wenn diese sich am nächsten zu der Position der Sonde befindet, basierend auf der Position der Sonde und dem Bild des Bedieners.

10. Verfahren gemäß einem oder mehreren der vorangegangenen Ansprüche, wobei die Anzeige eine erste Anzeige und eine zweite Anzeige umfasst, und
das Verfahren weiterhin das Anpassen eines Winkels zwischen der ersten Anzeige und der zweiten Anzeige umfasst,
wobei optional das Anpassen des Winkels zwischen der ersten Anzeige und der zweiten Anzeige umfasst: entweder
Anpassen des Winkels zwischen der ersten Anzeige und der zweiten Anzeige derart, dass ein Bildschirm der ersten Anzeige und ein Bildschirm der zweiten Anzeige sich in der Betrachtungsrichtung des Bedieners befinden; oder
Anpassen des Winkels zwischen der ersten Anzeige und der zweiten Anzeige derart, dass ein Bildschirm der ersten Anzeige sich in der Betrachtungsrichtung des Bedieners befindet, und der Bildschirm der zweiten Anzeige sich in der Betrachtungsrichtung des Objekts befindet.

11. Verfahren gemäß einem oder mehreren der vorangegangenen Ansprüche, das weiterhin umfasst:
Erfassen einer Geste des Bedieners in Bezug auf die sich an der zweiten Position befindende Anzeige; und
Durchführen einer Funktion der Ultraschalldiagnosevorrichtung gemäß der Geste des Bedieners, wobei das Erfassen der Geste des Bedieners optional umfasst:
Erfassen einer Geste des Bedieners mit Hilfe des Bildes des Bedieners, und wobei das Erfassen der Geste des Bedieners optional umfasst: Durchführen einer Funktion aus einer Menge von auf der Anzeige angezeigten Funktionen entsprechend einer Position, die von einer Hand des Bedieners angezeigt wurde, wenn basierend auf dem Bild des Bedieners bestimmt worden ist, dass die Hand des Bedieners und die Anzeige sich für einen vorgegebenen Zeitraum in einer vorgegeben Distanz befinden, oder
Erfassen einer Geste des Bedieners mit Hilfe einer an der Anzeige befestigten Gestenerfassungskamera, oder
Erfassen einer Geste oder Bewegung eines Auges des Bedieners mit Hilfe eines an der Anzeige oder einem Headset des Bedieners befestigten Augenbewegungssensors; und Durchführen einer Funktion der Ultraschalldiagnosevorrichtung gemäß der erfassten Geste oder der erfassten Bewegung des Auges des Bedieners.

12. Verfahren gemäß einem oder mehreren der vorangegangenen Ansprüche, das weiterhin das Empfangen einer von dem Bediener stammenden Eingabe zur Änderung der Anzeigeposition umfasst, und wobei die Eingabe zur Änderung der Anzeigeposition umfasst: eine Eingabe über einen mit der Ultraschalldiagnosevorrichtung verbundenen Fußschalter, eine Eingabe durch eine Stimme des Bedieners und/oder eine Eingabe durch eine Geste des Bedieners.

13. Verfahren gemäß einem oder mehreren der vorangegangenen Ansprüche, das weiterhin umfasst:
Empfangen einer Erfassungsendeingabe durch den Bediener oder Empfangen einer Eingabe des Bedieners, die Bilderfassung zu beenden; und
Ändern der Position der Anzeige von der zweiten Position zu der ersten Position gemäß der Erfassungsendeingabe.

14. Übergangsloses computerlesbares Speichermedium, auf dem ein Programm zur Ausführung des Verfahrens gemäß Anspruch 1 gespeichert ist.

15. Ultraschalldiagnosevorrichtung, die umfasst:
eine Bilderfassungseinheit zum Erhalten eines Bildes eines Bedieners der Ultraschalldiagnosevorrichtung, welcher eine Ultraschallbildgebung an dem Objekt mit Hilfe der Ultraschalldiagnosevorrichtung durchführt;
eine Bestimmungseinheit zum Bestimmen von wenigstens einer Betrachtungsrichtung und optional einer Position des Bedieners aus dem erhaltenen Bild des Bedieners;
eine Anzeige zum Anzeigen von Informationen über das Objekt und/oder eines Ultraschallbildes des Objekts; und
eine Steuereinheit zum Ändern von wenigstens einer der folgenden:
einer Ausrichtung von einem ersten Winkel zu einer zweiten Winkel, und
einer Position von einer ersten Position zu einer zweiten Position der mit der Ultraschalldiagnosevorrichtung verbundenen Anzeige gemäß wenigstens der bestimmten Betrachtungsrichtung und optional der bestimmten Position des Bedieners,
**dadurch gekennzeichnet, dass**
die Ultraschalldiagnosevorrichtung weiterhin umfasst:
ein Mittel zum Erhalten des Bildes eines Objektes;
ein Mittel zum Erhalten von Informationen über einen Abschnitt des Objekts, der erfasst werden wird;
wobei die Bestimmungseinheit ebenfalls die Position des Abschnitts des Objekts aus dem Bild des Objekts bestimmt; und
wobei die Steuereinheit ebenfalls eine Position der Anzeige von einer dritten Position in die erste Position ändert basierend auf der bestimmten Position des Abschnitts des Objekts.

## Revendications

1. Procédé d'exploitation d'un dispositif d'affichage d'un appareil échographique de diagnostic possédant au moins un dispositif d'affichage, le procédé comprenant :
l'obtention d'une image d'un objet,
l'obtention d'informations concernant une partie de l'objet destinée à des prises de vue au moyen de l'appareil échographique de diagnostic,
la détermination de la position de la partie de l'objet à partir de l'image de l'objet,
la modification de la position du dispositif d'affichage compte tenu de la position déterminée de la partie de l'objet,
l'obtention d'une image d'un opérateur de l'appareil échographique de diagnostic qui réalise l'échographie de l'objet au moyen de l'appareil échographique de diagnostic,
la détermination d'au moins un sens de visée et éventuellement de la position de l'opérateur à partir de l'image de l'opérateur obtenue, et
la modification
de l'orientation, d'un premier angle à un deuxième angle, et
de la position, d'une première position à une deuxième position
du dispositif d'affichage branché sur l'appareil échographique de diagnostic conformément, au moins, au sens de visée déterminée et éventuellement à la position de l'opérateur déterminée.

2. Procédé selon la revendication 1, dans lequel la modification de la position du dispositif d'affichage comprend :
la détermination d'une plage de visée de l'opérateur compte tenu du sens de visée déterminé et éventuellement de la position de l'opérateur déterminée, et
la modification de l'orientation et/ou de la position du dispositif d'affichage, un écran du dispositif d'affichage étant situé dans la plage de visée de l'opérateur déterminée ;
dans lequel éventuellement la modification de la position du dispositif d'affichage comprend la détermination d'une position, située dans la plage de visée de l'opérateur et dans un espace au sein duquel le dispositif d'affichage est déplaçable, en tant que deuxième position.

3. Procédé selon la revendication 1 ou 2, dans lequel la détermination du sens de visée de l'opérateur comprend :
la détermination de la posture de l'opérateur compte tenu de l'image de l'opérateur, et
la détermination du sens de visée de l'opérateur au moyen de la posture de l'opérateur déterminée ;
dans lequel éventuellement, en outre, la détermination de la posture de l'opérateur comprend la détermination de la posture de l'opérateur compte tenu de la forme et/ou de la relation de position entre une partie de tête et une partie de corps de l'opérateur incluses dans l'image de l'opérateur.

4. Procédé selon la revendication 1, 2 ou 3, dans lequel l'image de l'opérateur comprend en outre un plan de support supportant l'objet, et
la détermination au moins du sens de visée et/ou la détermination de la position de l'opérateur comprend la détermination d'une relation de position entre l'opérateur et le plan de support compris dans l'image de l'opérateur.

5. Procédé selon l'une ou plusieurs des revendications précédentes, dans lequel la modification de la position du dispositif d'affichage comprend la détermination de la deuxième position, et la détermination de la deuxième position comprend la détermination d'une relation de position entre l'appareil échographique de diagnostic et l'opérateur.

6. Procédé selon l'une ou plusieurs des revendications précédentes, dans lequel :
l'obtention de l'image de l'opérateur comprend l'obtention d'une image en profondeur de l'opérateur, et
la détermination au moins du sens de visée et éventuellement de la position de l'opérateur comprend la détermination du sens de visée et éventuellement de la position de l'opérateur compte tenu de l'image en profondeur de l'opérateur ;
dans lequel l'obtention de l'image en profondeur comprend une ou plusieurs des opérations suivantes :
l'obtention d'une image en profondeur de l'opérateur au moyen d'un dispositif de prise de vue en profondeur, et
l'obtention d'images de l'opérateur au moyen d'au moins deux dispositifs de prise de vue en couleur et l'obtention d'une image en profondeur de l'opérateur par application d'une stéréocorrespondance aux images de l'opérateur obtenues au moyen des au moins deux dispositifs de prise de vue en couleur.

7. Procédé selon l'une ou plusieurs des revendications précédentes, dans lequel la modification de la position du dispositif d'affichage comprend la détermination d'une trajectoire de déplacement, d'un premier angle à un deuxième angle, et/ou de la première position à la deuxième position, compte tenu d'informations concernant la position d'une personne et/ou d'un objet inclus dans l'image de l'opérateur, et
dans lequel éventuellement la détermination de la trajectoire de déplacement comprend la détermination de la trajectoire de déplacement, d'un premier angle à un deuxième angle et/ou de la première position à la deuxième position, de manière que le dispositif d'affichage n'entre pas en collision avec un obstacle, la personne et/ou l'objet inclus dans l'image de l'opérateur.

8. Procédé selon l'une ou plusieurs des revendications précédentes, dans lequel la modification de la position du dispositif d'affichage comprend une modification, d'une première trajectoire de déplacement à une deuxième trajectoire de déplacement, du dispositif d'affichage après détermination qu'il se trouve un obstacle sur la première trajectoire de déplacement du dispositif d'affichage,
dans lequel aucun obstacle ne se trouve sur la deuxième trajectoire de déplacement, et
dans lequel la première et la deuxième trajectoire de déplacement sont des trajectoires permettant un déplacement entre la première position du dispositif d'affichage et la deuxième position du dispositif d'affichage.

9. Procédé selon l'une ou plusieurs des revendications précédentes, dans lequel la détermination au moins du sens de visée et éventuellement de la position de l'opérateur comprend la détermination du fait que la personne est l'opérateur lorsque cette personne est la plus proche de l'emplacement de la sonde de l'appareil échographique de diagnostic lorsqu'une pluralité de personnes sont comprises dans l'image de l'opérateur,
dans lequel éventuellement la détermination de la personne qui est la plus proche de l'emplacement de la sonde de l'appareil échographique de diagnostic comme étant l'opérateur comprend :
la détermination de la position de la sonde par infrarouges ou par une communication de courte portée, et
la détermination de la personne qui est la plus proche de la position de la sonde, compte tenu de la position de la sonde et de l'image de l'opérateur, comme étant l'opérateur.

10. Procédé selon l'une ou plusieurs des revendications précédentes, dans lequel le dispositif d'affichage comprend un premier dispositif d'affichage et un deuxième dispositif d'affichage, et
le procédé comprenant en outre le réglage d'un angle entre le premier dispositif d'affichage et le deuxième dispositif d'affichage,
dans lequel éventuellement le réglage de l'angle entre le premier dispositif d'affichage et le deuxième dispositif d'affichage comprend soit :
le réglage de l'angle entre le premier dispositif d'affichage et le deuxième dispositif d'affichage de manière qu'un écran du premier dispositif d'affichage et un écran du deuxième dispositif d'affichage soient situés dans le sens de visée de l'opérateur, soit
le réglage de l'angle entre le premier dispositif d'affichage et le deuxième dispositif d'affichage de manière qu'un écran du premier dispositif d'affichage soit situé dans le sens de visée de l'opérateur et que l'écran du deuxième dispositif d'affichage soit situé dans un sens de visée de l'objet.

11. Procédé selon l'une ou plusieurs des revendications précédentes, comprenant en outre :
la détection d'un geste de l'opérateur par rapport au dispositif d'affichage situé dans la deuxième position, et
la réalisation d'une fonction de l'appareil échographique de diagnostic étant donné le geste de l'opérateur ;
dans lequel la détection du geste de l'opérateur comprend éventuellement :
la détection d'un geste de l'opérateur au moyen de l'image de l'opérateur, ladite détection du geste de l'opérateur comprenant éventuellement la réalisation d'une fonction étant donné une position indiquée par la main de l'opérateur parmi des fonctions affichées sur le dispositif d'affichage lorsqu'il a été déterminé, compte tenu de l'image de l'opérateur, que la main de l'opérateur et le dispositif d'affichage sont situés à une distance prédéfinie pendant un temps prédéfini, ou
la détection d'un geste de l'opérateur au moyen d'une caméra de détection de gestes rattachée au dispositif d'affichage, ou
la détection d'un geste ou d'un mouvement de l'oeil de l'opérateur au moyen d'un détecteur de suivi oculaire rattaché au dispositif d'affichage ou rattaché à un casque de l'opérateur ; et la réalisation d'une fonction de l'appareil échographique de diagnostic étant donné le geste ou le mouvement de l'oeil de l'opérateur détecté.

12. Procédé selon l'une ou plusieurs des revendications précédentes, comprenant en outre la réception d'une entrée de modification de position du dispositif d'affichage en provenance de l'opérateur, et l'entrée de modification de position du dispositif d'affichage comprend une entrée réalisée par le biais d'un interrupteur à pédale branché sur l'appareil échographique de diagnostic, une entrée vocale de l'opérateur, et une entrée réalisée par le biais d'un geste de l'opérateur.

13. Procédé selon l'une ou plusieurs des revendications précédentes, comprenant en outre :
la réception d'une entrée de fin de prise de vue émanant de l'opérateur ou la réception d'une entrée provenant de l'opérateur pour terminer la prise de vue, et
la modification de la position du dispositif d'affichage, de la deuxième position à la première position conformément à l'entrée de fin de prise de vue.

14. Support de stockage non transitoire lisible par ordinateur dans lequel est enregistré un programme permettant d'exécuter le procédé selon la revendication 1.

15. Appareil échographique de diagnostic comprenant :
une unité d'obtention d'image permettant d'obtenir une image d'un opérateur de l'appareil échographique de diagnostic réalisant une échographie d'un objet au moyen de l'appareil échographique de diagnostic,
une unité de détermination permettant de déterminer au moins un sens de visée et éventuellement la position de l'opérateur à partir de l'image de l'opérateur obtenue,
un dispositif d'affichage permettant d'afficher des informations concernant l'objet et/ou une image échographique de l'objet, et
une unité de commande destinée à modifier
l'orientation, d'un premier angle à un deuxième angle, et/ou
la position, d'une première position à une deuxième position
du dispositif d'affichage branché sur l'appareil échographique de diagnostic conformément, au moins, au sens de visée déterminée et éventuellement à la position de l'opérateur déterminée,
**caractérisé en ce que**
l'appareil échographique de diagnostic comprend en outre :
un moyen permettant d'obtenir une image d'un objet, et
un moyen permettant d'obtenir des informations concernant une partie de l'objet destinée à des prises de vue ;
dans lequel l'unité de détermination détermine également la position de la partie de l'objet à partir de l'image de l'objet, et
dans lequel l'unité de commande modifie également la position du dispositif d'affichage, d'une troisième position à la première position, compte tenu de la position déterminée de la partie de l'objet.
